# EUROPEAN PATENT APPLICATION

(11) **EP 2 949 339 A1**
(43) Date of publication of application: **02.12.2015**
(21) Application number: 14170491.6
(22) Date of filing: 29.05.2014
(51) Int. Cl.: A61K 39/00, A61K 31/661, A61K 35/14, A61P 35/00

(54) **Agonist of CD1c-restricted T cells and uses thereof**

(71) Applicant: Ospedale San Raffaele S.r.l., 20132 Milano (IT); Fondazione Centro San Raffaele S.r.l., 20132 Milano (MI) (IT); Universitätsspital Basel Rechtsdienst /IPR, 4031 Basel (CH)
(72) Inventor: Casorati, Giulia, 20132 Milano (MI) (IT); Dellabona, Paolo, 20132 Milano (MI) (IT); De Lalla, Claudia, 20132 Milano (MI) (IT); De Libero, Gennaro, 4103 Bottmingen (CH); Lepore, Marco, 4057 Basel (CH)
(74) Representative: Capasso, Olga

(57) **Abstract**

An agonist of CD1c-restricted T cells or an agonist-specific-CD1c-restricted T cell, their medical and diagnostic uses and compositions comprising the same. In particular the agonist is a lipid antigen.

## Description

### FIELD OF THE INVENTION

The invention relates to an agonist of CD1c-restricted T cells or an agonist-specific-CD1c-restricted T cell, their medical and diagnostic uses and compositions comprising the same. In particular the agonist is a lipid antigen.

### BACKGROUND OF THE INVENTION

CD1-restricted T lymphocytes recognize lipid antigens presented by the nonpolymorphic, MHC class I-related family of CD1 molecules (Porcelli and Modlin, 1999). CD1-restricted T cells can respond to lipid antigens derived from microbial cells and may exert protective roles during host infection (Amprey et al., 2004; Gilleron et al., 2004; Kinjo et al., 2005; Montamat-Sicotte et al., 2011; Moody et al., 2000; Moody et al., 2004; Sriram et al., 2005; Wu et al., 2005). A striking characteristic of many CD1-restricted T cells is auto-reactivity against different types of antigen presenting cells (APCs) even in the absence of microbial antigens, implying that they can also recognize endogenous self-lipid molecules (Dellabona et al., 1993; Mattner et al., 2005; Vincent et al., 2005). Auto-reactive T cells recognize different types of self-lipids present in cell membranes and synthesized within different cellular compartments (De Libero et al., 2005; Gumperz et al., 2000; Shamshiev et al., 1999; Shamshiev et al., 2000; Wu et al., 2003). CD1a- and CD1c-autoreactive T cells are relatively abundant among circulating T cells in healthy individuals (de Jong et al., 2010; de Lalla et al., 2011) and might become activated by host antigens in autoimmune diseases and cancer. Lipid-specific T cells can control cancer cell growth in mouse models (Berzofsky and Terabe, 2009) as well as in human patients (Dhodapkar and Richter, 2011; Metelitsa, 2011), but it remains unknown whether they recognize unique lipids expressed by tumor cells.

Acute leukemia comprises a heterogeneous group of hematological disorders characterized by blood and bone marrow accumulation of immature and abnormal cells derived from hematopoietic precursors (Pui et al., 2004; Rubnitz et al., 2008).

Current therapy for acute leukemia is based on poly-chemotherapy and allogeneic hematopoietic stem cell transplantation (HSCT). A major cause of treatment failure and area of substantial unmet need in HSCT is post-transplant re-growth of residual leukemia blasts that survive the conditioning regimen (Wingard et al., 2011). Donor-derived T cells transferred into patients may induce a beneficial graft versus leukemia (GVL) reaction capable of maintaining remission (Kolb, 2008), but grafted T cells are also capable of killing patient cells in non-hematopoietic tissues to induce detrimental graft versus host disease (GVHD) (Socie and Blazar, 2009). A promising therapeutic strategy is the selective targeting of T cell responses against malignant hematopoietic cells, while maintaining hematopoietic capacity among grafted cells and preserving organ functions in recipient patients (Kolb, 2008). Since CD1 molecules are both nonpolymorphic and preferentially expressed by mature hematopoietic cells (Brigl and Brenner, 2004; Porcelli and Modlin, 1999), targeting tumor-associated lipid antigens presented by CD1 molecules might provide opportunities to improve the efficacy of HSCT. Immune recognition of tumor-associated lipid antigens may also complement ongoing anti-tumor responses mediated by protein antigens.

### SUMMARY OF INVENTION

T-cells that recognize self-lipids presented by CD1c are frequent in the peripheral blood of healthy individuals and kill transformed hematopoietic cells, but little is known about their antigen specificity and potential anti-leukemia effects. In the present invention, the inventors have identified the novel self-lipid antigen that stimulates CD1c auto-reactive T cells to destroy tumor cell lines and primary human leukemia cells. The inventors report that both group 1 CD1 molecules and a novel class of tumor-associated lipids are broadly expressed by different types of acute leukemia. In addition to killing CD1c+ leukemia cell lines and primary blasts *in vitro,* the CD1c-restricted T cells also displayed therapeutic efficacy in a mouse xenograft model of human leukemia. The inventors'findings provided proof-of-concept evidences that T cell responses against lipids accumulated in acute leukemia could be exploited for leukemia immunotherapy.

The inventors report that CD1c self-reactive T-cells recognize a novel class of self-lipids, identified as methyl-lysophosphatidic acids, which are accumulated in leukemia cells. Primary acute myeloid and B-cell acute leukemia blasts express CD1 molecules. Methyl-lysophosphatidic acid-specific T-cells efficiently kill CD1c+ acute leukemia cells, poorly recognize non-transformed CD1c-expressing cells, and protect immune-deficient mice against CD1c+ human leukemia cells. The identification of immunogenic self-lipid antigens accumulated in leukemia cells and the observed leukemia control by lipid-specific T-cells *in vivo* provide a new conceptual framework for leukemia immune surveillance and possible immunotherapy.

In the present invention the following abbreviations were used: AML, acute myeloid leukemia; B-ALL, B-cell acute lymphoblastic leukemia; COSY, correlation spectroscopy; Da, Dalton; GVHD, graft versus host disease; GVL, graft versus leukemia disease; HMBC, heteronuclear multiple bond correlation; HPLC, high-performance liquid chromatography; HRMS, high-resolution mass spectrometry; HSC, hematopoietic stem cells; HSCT, hematopoietic stem cells transplantation; HSQC, heteronuclear single quantum coherence spectroscopy correlation; LAIP, leukemia associated immune phenotype; LC-MS-MS, liquid chromatography-tandem mass spectrometry; MS, mass spectrometry; LPA, lysophosphatidic acid; LPC, lysophosphatidylcholine; LPE, lysophosphatidylethanolamine; LPM, lysophosphomethanol; m/z, mass/charge; mLPA, methyl-lysophosphatidic acid; NMR, nuclear magnetic resonance; NOD/scid, nonobese diabetic/severe combined immunodeficiency; NOESY, nuclear Overhauser effect spectroscopy; NSG, NOD/scid/common γ chain; ppm, parts per million; CD1c, soluble CD1c; T-ALL, T-cell acute lymphoblastic leukemia; UPLC, ultraperformance liquid chromatography.

Therefore it is an embodiment of the invention an agonist of CD1c-restricted T cells or an agonist-specific-CD1c-restricted T cell for use in the treatment and/or prevention of a hematological disorder.

Preferably the agonist is a self-lipid antigen. Still preferably the agonist is a lysoglycerolphosphate molecule.

Yet preferably the agonist is a Methyl-lysophosphatidic acid (mLPA), preferably C16-methyl-lysophosphatidic acid (C16-mLPA) or C18-methyl-lysophosphatidic acid (C18-mLPA).

In a preferred embodiment the agonist is used as a vaccine.

In a further embodiment the hematological disorder is characterized by blood and bone marrow accumulation of immature and abnormal cells derived from hematopoietic precursors. Preferably the hematological disorder is acute leukemia, preferably primary acute myeloid leukemia or B-cell acute leukemia.

A further embodiment of the invention is a pharmaceutical composition comprising an effective amount of at least one agonist as defined above and pharmaceutical acceptable vehicle for use in the treatment and/or prevention of a hematological disorder.

In a preferred embodiment the pharmaceutical composition further comprises an effective amount of at least another therapeutic agent.

Preferably the other therapeutic agent is any peptide or whole protein antigen derived from cancer cells that is already in clinical use as component of cancer vaccines. More preferably the other therapeutic agent is selected from the group of: NY-ESO1, MAGEA3, WT-1, MelanA/MART1, NA-17.

In a further embodiment the agonist or said T cell of the invention is combined with another therapeutic intervention. Preferably the therapeutic intervention is allogenic hematopoietic stem cell transplantation.

The stem cell used is obtained or obtainable by methods known to the skilled person in the art and that do not involve the destruction of any embryo.

A further embodiment of the invention is the use of an agonist as defined above to stimulate CD1c-restricted T cell.

A further embodiment of the invention is a CD1c-restricted T cell stimulated by an agonist as above for use in the treatment and/or prevention of a hematological disorder.

A further embodiment of the invention is method for diagnosing and/or monitoring the course of a hematological disorder and/or for assessing the efficacy of a treatment of a hematological disorder in a subject comprising measuring the amount of the agonist as defined above in a biological sample obtained from the subject.

A further embodiment of the invention is method for diagnosing and/or monitoring the course of a hematological disorder and/or for assessing the efficacy of a treatment of a hematological disorder in a subject comprising measuring the number and/or the frequency of an agonist-specific-CD1c-restricted T cell as defined above in a biological sample obtained from the subject.

Preferably the biological sample is selected from the group consisting of: blood or bone marrow biopsy.

A further embodiment of the invention is a method of treating and/or preventing a hematological disorder comprising administering to a subject in need thereof an effective amount of the agonist of CD1c-restricted T cells or of the agonist-secific-CD1c-restricted T cell as defined above or of the pharmaceutical composition as defined above.

In the present invention an agonist is a molecule able to stimulate CD1c-restricted T cells.

A T cell stimulating compound is a compound that can either increase proliferation, or upregulate activation markers, elicit cytokine production or elicit citotoxicity by CD1c-restricted T-cells when introduced to the immune system..

By "stimulated" it is intended a state that is different from baseline, whereby CD1c-restricted T lymphocyte either enter cell division and increase their number, or upregulate on the cell surface the expression of known activation markers, or express cytokines that correlate with their functions, or kill CD1c-expressing target cells.

A self-lipid antigen means a lipid that is produced by the metabolic machinery intrinsic of each cell of a given organism, which is recognized by T or B lymphocytes autologous to the same organism.

The present invention utilizes the concept that a CD1c-restricted T cells agonist, such as an antigen elicits or boosts an immune response to the non-peptide antigen. Hence, the invention relates to an immunogenic composition or vaccine composition comprising a CD 1 c-restricted T cells agonist, as well as methods of use thereof.

The invention pertains to an immunogenic composition comprising at least one CD1c-restricted T cells agonist, wherein the agonist elicits a CD1c-restricted response. An immunogenic composition refers to a composition that contains this agonist and can produce or boost an immune response through the CD1c-antigen presenting pathway, as described herein. A CD1c-restricted response is a response in which the CD1c-antigen presenting pathway is utilized, and can be independent of the MHC-antigen presenting pathway.

To "boost" the immune response refers to enhancing the immune response of an individual or mammal, as compared to the immune response prior to administration of the composition of the present invention. An increase or enhancement of various immunological parameters, as compared to a baseline (e. g., prior to administration of the composition) occurs.

To "elicit" an immune response is to create an immune response to an antigen, sufficient to confer a protective or immune enhancing effect against the CD1c-restricted T cells agonist. In particular, T-cell proliferation occurs, T-cell cytokines are produced and/or antibodies are made in response to the antigen. Various immunological parameters can be measured to assess the enhancement or elicitation of an immune response, such as an antibody response to the antigen (e. g., level or titer), cytotoxic T-lymphocyte response, helper T-cell response, T-cell proliferative response, or a T-cell modulated cytokine response (e. g., a cytokine that is produced by the T-cell response). One or more of these immunological parameters increase in response to the CD1c-restricted T cells agonist composition as compared to the responses prior to administration of the composition.

One can measure a baseline level of the various immunological parameters prior to administering the compound and then reassess those levels after administering the composition. An increase in one or more of the immunological parameters occurs, as compared to the baseline. The immunological parameters can also be measured against a control or levels from a control population to which the composition has already been administered (e. g., positive control) or to a population which has not been exposed to the composition (e. g., a negative control). These immunological parameters can be measured utilizing methods known in the art. Accordingly, the present invention includes methods and vaccine compositions to elicit a protective effect, as well as methods and immunogenic compositions for boosting or enhancing the immune response to the CD1c-restricted T cells agonist.

In addition to the methods and compositions that utilize the CD1c-restricted T cells agonist composition, the present invention further includes eliciting an immune enhancing effect to peptide antigens. Several non-peptide antigens can be used to combat one or more diseases, and can be combined with peptide based vaccines to produce a more effective and complete vaccine composition. The non-peptide antigen can be combined with one or more peptide antigens from the same organism or tumor cell to provide a composition which confers a protective or immune enhancing effect against a multitude of antigens, including peptide and non-peptide antigens. Hence, the present invention includes vaccine or immunogenic compositions that comprise not only the non-peptide or lipid antigen and adjuvant, but also a peptide based antigen. The administration of the CD1c-restricted T cells stimulating compound (agonist) with other therapeutic agents can occur simultaneously or sequentially in time. The T-cell stimulating compound can be administered before, after or at the same time as the other therapeutic agent. Thus, the term "coadministration" is used herein to mean that the CD1c-restricted T cells agonist and any other therapeutic agent will be administered at times to achieve the protective or immune enhancing effect. The methods of the present invention are not limited to the sequence in which the agonist and other agent are administered, so long as the other agent is administered close enough in time to produce the desired effect, e. g., boosting or eliciting a CD1c-restricted T cells response or a MHC-restricted T cell response. In a preferred embodiment, the administration of the agonist/other therapeutic agent occurs simultaneously. The methods also include co-administration with other drugs that aid in eliciting or boosting an immunogenic response (e. g., cytokines such as IL-2, GM-CSF, and IL-12 and/or antibodies). A non-peptide antigen refers to an antigen in which the whole or a portion thereof is not a peptide, e. g., does not contain two or more amino acids in which a carboxyl group of one is united with an amino group of the other, with elimination of a water molecule. A lipid antigen can be obtained from a variety of biological sources, or can be synthesized. Such antigens can be isolated or synthesized by standard methods known to those skilled in the art, so that characteristics which allow the non-peptide antigen to be presented in the context of the CD1-antigen presenting pathway remain. These nonpeptide or lipid antigens can be isolated from a variety of biological sources including bacteria (e. g., gram negative or gram positive), parasites, plants, fungus, or other mammalian origins. The non-peptide antigen of the invention can be present in the form of the whole, but inactivated bacteria or parasite; and/or bacterial or parasitic portions thereof which retain their antigenicity. These lipid antigens are found in the cell wall of bacteria, parasites and/or fungi. For example, lipid antigen components of Mycobacteria can be isolated by standard methods described in Goren, M. B. and Brennan, P. J., Mycobacterial Lipids : Chemistzy and Biologic Activities in Tuberculosis, 1979, In Tuberculosis; ed. G. P. Youmans, pp. 63-193, (Philadelphia, WB Saunders 1979), and Hunter S. W., et al., J. Biol. Chem. 261: 12345-12531 (1986).

Lipid fractions can be prepared from cultures of bacteria (e. g., Mycobacterium tuberculosis). Lipid fractions can be obtained using an alcohol mixture such as acetone or methanol, or chloroform. Extraction with chloroform or methanol separates the organic extract (O/E) from insoluble, delipidated cell wall fractions. The O/E is placed on a silicone column and eluded with chloroform, acetone, and then methanol, which yields three purified fractions. The chloroform fraction contains triglycerides and free fatty acids. The acetone fraction contains glycolipid, and the methanol fraction contains various phospholipids such as phosphatidyl inositol, phosphatidyl ethanolamine and phosphatidyl glycerol. Accordingly, a lipid fraction such as a chloroform, acetone, or methanol fraction can be used to isolate a lipid antigen for the present invention.

An "isolated" lipid antigenic fraction refers to a fraction containing a substantially purified amount of lipid molecules or antigens (e. g., greater than 80%, 85%, 90%, or 95%). Accordingly, the present invention includes immunogenic compositions or vaccine compositions that contain a portion of a organism (e. g., contains a CD1c or lipid antigen), or the entire organism which has been altered to provide a protective or immune enhancing response without producing infection of the naturally occurring, live organism, e. g., without causing disease. Examples of these vaccines are live organism attenuative vaccines, killed organism vaccines, subunit vaccines, or toxoid vaccines. The invention can utilize synthetic derived antigens that utilize the CD1c- restricted pathway. A synthetic antigen, in accordance with this invention, is an antigen which is not naturally occurring in an organism. A synthetic antigen may be one that chemically synthesized, or parts thereof synthesized by combining elements, molecules or compounds. Further, a synthetic antigen can be constructed by combining two or more components, one or more of which is isolated or derived from an organism, thus producing a hybrid or chimeric antigen. A synthetic antigen, comprising a lipid moiety and a hydrophilic moiety which is chemically synthesized, can be presented to a T cell by an APC in association with CD1 molecules through the CD1 antigen presenting pathway. The composition of the invention may also include adjuvants. An adjuvant is a substance which enhances the immunogenic response to an antigen. In particular, adjuvants are generally considered to be in one of two categories: 1)"vehicles" that help to transport and retain antigens in lymphoid tissues, or 2)"immuno-modulates" that stimulate locally secreted cytokines. Adjuvants can composed of bacteria or bacterial products which contribute to the immunogenicity of the antigen. Examples of adjuvants are mineral salt adjuvants (alum or calcium based adjuvant), Incomplete or Complete Freund's Adjuvant, Bacille Calmette-Guerin (BCG) adjuvant, block polymer adjuvant (Titermax), cholera toxin (Cholera toxin-B-subunits, enterotoxin (LT) mutants), cytokines, CPG motif containing adjuvant, oil/water emulsion adjuvant (oil and water; water and oil; water, oil and water adjuvants), MF-59 adjuvants, LeIF adjuvants (e. g., protein based), liposome adjuvant, ISCOM adjuvant, Monophosphoryl A adjuvant (MPL), biodegradable microsphere adjuvant, muramyl dipeptide adjuvant, polyphosphozine adjuvant, and saponin adjuvant (QS-7, QS-17, QS-18, or QS-21). Preferably, the compositions comprise two or more adjuvants (e. g., QS-21 and MPL).

Other lipid based vaccines are encompassed by the present invention.

Individuals having diseases, such as cancer and infectious diseases, can be vaccinated using a lipid/adjuvant composition. Cancers include melanoma, breast cancer, prostate cancer, lung cancer, colo-rectal cancer, chronic leukemia, acute leukemia, lymphoma. Lipid based antigens from tumors can be used as the non-peptide antigen for the present invention. Similarly, infectious diseases such as pneumonia, meningitis, otitis, diarrhea.

The composition of the present invention can be administered with or without a carrier. The terms "pharmaceutically acceptable carrier" or a "carrier" refer to any generally acceptable excipient or drug delivery composition that is relatively inert and non-toxic. Exemplary carriers include sterile water, salt solutions (such as Ringer's solution), alcohols, gelatin, talc, viscous paraffin, fatty acid esters, hydroxymethylcellulose, polyvinyl pyrolidone, calcium carbonate, carbohydrates such as lactose, sucrose, dextrose, mannose, albumin, starch, cellulose, silica gel, polyethylene glycol (PEG), dried skim milk, rice flour, magnesium stearate, and the like. Suitable formulations and additional carriers are described in Remington's Pharmaceutical Sciences, (17th Ed., Mack Pub. Co., Easton, PA), the teachings of which are incorporated herein by reference in their entirety. Such preparations can be sterilized and, if desired, mixed with auxiliary agents, e. g., lubricants, preservatives, stabilizers, wetting agents, emulsifiers, salts for influencing osmotic pressure, buffers, coloring, and/or aromatic substances and the like which do not deleteriously react with the active compounds. They can also be combined where desired with other active substances, e. g., enzyme inhibitors, to reduce metabolic degradation. A carrier (e. g., a pharmaceutically acceptable carrier) is preferred, but not necessary to administer the compound.

The present invention provides a variety of pharmaceutical compositions. Such compositions comprise a therapeutically (or prophylactically) effective amount of an immunogenic or vaccine compositions, including a CD1c-restricted T cell agonist, adjuvant, peptide antigen and/or a carrier. The composition, if desired, can also contain minor amounts of wetting or emulsifying agents, or pH buffering agents, or preservatives. Typical preservatives can include, potassium sorbate, sodium metabisulfite, methyl paraben, propyl paraben, thimerosal, etc.

The composition can be a liquid solution, suspension, emulsion, tablet, pill, capsule, sustained release formulation, or powder. The method of administration can dictate how the composition will be formulated. For example, the composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. The carrier can be added to the composition at any convenient time. In the case of a lyophilized vaccine, the carrier can, for example, be added immediately prior to administration. Alternatively, the final product can be manufactured with the carrier. The immunogenic vaccine compositions of the invention can be administered intravenously, parenterally, intramuscular, subcutaneously, orally, nasally, topically, by inhalation, by implant, by injection, or by suppository. The composition can be administered in a single dose or in more than one dose (e. g., boosted) over a period of time to confer the desired effect. For enteral or mucosal application (including via oral and nasal mucosa), particularly suitable are tablets, liquids, drops, suppositories or capsules. A syrup, elixir or the like can be used wherein a sweetened vehicle is employed. Topical application can also be used for example, in intraocular administration. Alternative methods of administration can include an immune-stimulating complex (ISCOM) as described in U. S. patent No. 4,900,549 (or European Patent Publication No. 0 604 727 A1 (Publ. July 6,1994). In addition to liposomes, viral vectors, microspheres, and microcapsules are available and can be used. For parenteral application, particularly suitable are injectable, sterile solutions, preferably oily or aqueous solutions, as well as suspensions, emulsions, or implants, including suppositories. In particular, carriers for parenteral administration include aqueous solutions of dextrose, saline, pure water, ethanol, glycerol, propylene glycol, peanut oil, sesame oil, polyoxyethylene-polyoxypropylene block polymers, and the like. Ampules are convenient unit dosages. Methods of administration will vary in accordance with the type of disorder and microorganism sought to be controlled or eradicated. The dosage of the vaccine will be dependent upon the amount of antigen, its level of antigenicity, and the route of administration. A person of ordinary skill in the art can easily and readily titrate the dosage for an immunogenic response for each antigen-adjuvant complex and method of administration.

The actual effective amounts of compound or drug can vary according to the specific composition being utilized, the mode of administration and the age, weight and condition of the patient, for example. As used herein, an effective amount of the drug is an amount of the drug which elicits or boosts an immune response to the non-peptide antigen. Dosages for a particular patient can be determined by one of ordinary skill in the art using conventional considerations, (e. g. by means of an appropriate, conventional pharmacological protocol).

The present invention also relates to kits comprising at least one CD1c-restricted T-cell stimulating compound (agonist/antigen). As described herein, the antigen can be obtained or isolated from at least one bacteria and/or parasite, or any other organism containing a lipid-based antigen. The antigen can also be a tumor associated (e. g., cancer) antigen.

The kit can comprise various adjuvants as described herein. The determination of optimum dosages for a particular patient is well known to one skilled in the art.

As is common practice, the compositions are normally accompanied by written or printed instructions for use in the treatment in question.

The present invention will be illustrated by means of non-limiting examples referring to the following figures:

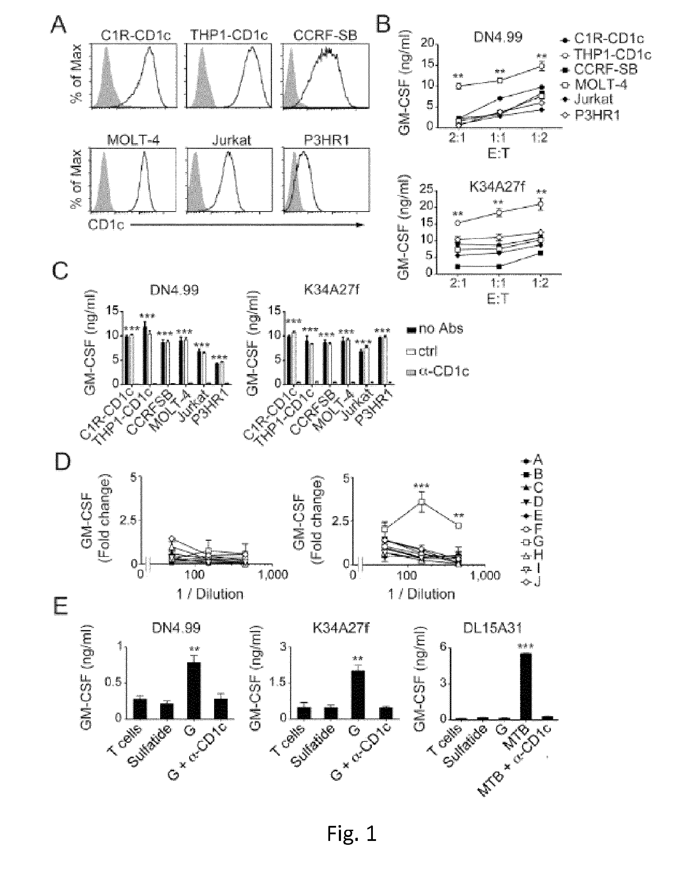

**Figure 1****. Recognition of leukemia cell lines and leukemia-derived lipids by CD1c auto-reactive T cells.** (A) Flow cytometry detection of CD1c surface protein in C1R and THP1 cell lines transfected with CD1c cDNA, and other leukemia cell lines constitutively expressing CD1c. Grey histograms represent staining with isotype-matched control mAbs. (B) Recognition of CD1c-expressing leukemia cell lines by CD1c self-reactive T cell clones DN4.99 (upper panel) and K34A27f (lower panel) at the indicated effector: target (E:T) ratios. T cell activation was assessed by measurements of GM-CSF release. (C) Anti-CD1c blocking mAbs (α-CD1c) inhibit the recognition of CD1c-expressing leukemia cell lines by DN4.99 (left panel) and K34A27f (right panel). T cell reactivity in absence of Abs (no Abs) and in presence of matched isotype control mAbs (ctrl) is shown. T cell activation was evaluated my measuring GM-CSF release. (D) Fractions (A-J) of THP1 lipids extracted from the organic phase (left panel) and aqueous phase (right panel) were used to stimulate DN4.99 T cells in the presence of fixed THP1-CD1c cells. T cell activation was assessed by measurement of GM-CSF release and expressed as fold change over background. (E) CD1c self-reactive T cell clones DN 4.99 (left panel), and K34A27f (middle panel), or CD1c-restricted *M. tuberculosis-specific* DL15A31 T cells (right panel) were cultured either alone (T cells) or in the presence of plastic-bound sCD1c molecules loaded with either Sulfatide, fraction G from aqueous extraction (G) or *M. tuberculosis* sonicate (MTB) with or without anti-CD1c blocking mAbs (α-CD1c). T cell activation was assessed by measurement of GM-CSF release. All data are represented as mean ± SD. Results are representative of three (B), three (C), seven (D) and three (E) independent experiments. **P < 0.01, *** P < 0.001, determined by two-tailed Student's *t*-test. P values in panels (C) refer to both groups "no Abs" and "ctrl" individually compared to the group "α-CD1c".

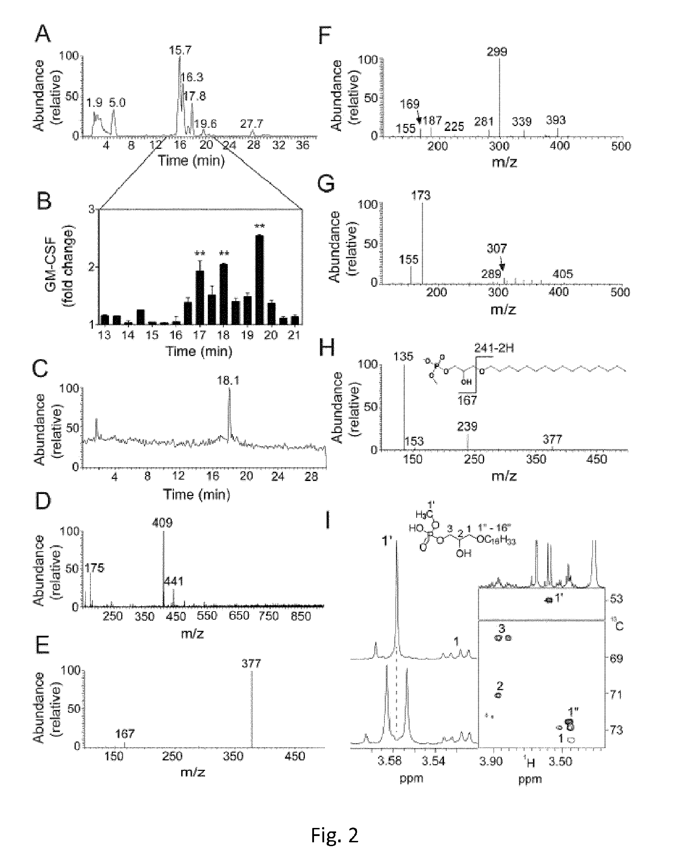

**Figure 2****. Identification of mLPA as a CD1c-restricted T cell antigen.** (A) LC-MS total ion profile of fraction G derived from aqueous extract. (B) Activation of DN4.99 T cells by fixed THP1-CD1c cells in the presence of HPLC-MS lipid subfractions (min 13-21) derived from fraction G. GM-CSF levels are expressed as fold change over background. Bars represent SD. **P < 0.01, determined by two-tailed Student's *t*-test. Data are representative of three independent experiments. (C) Profile of LC-MS re-chromatography of the active sub-fraction collected at 17 min. (D) Mass spectrum of the compound eluted at 18.1 min, showing a single molecular species with m/z 409 in negative ion mode. (E) MS2 spectrum of ion m/z 409. (F) MS2 spectrum of ion m/z 411 from fraction G as analyzed in positive mode. The presence of a methylated phosphate head group was confirmed by the detection of a major peak at m/z 299 (due to the loss of 112Da from parent ion m/z 411), and presence of ions at m/z 187 and 169. A low intensity ion at m/z 225 was attributable to a hexadecyl-carbocation, which supported the existence of a C16 alkyl chain (as predicted by the negative mode analysis). (G) MS2 spectrum of the unmodified lysophosphatidic acid 1-O-9-(Z)-octadecenyl-sn-glycero-3-phosphate. The spectrum showed signature ions for a phosphate head at m/z 155 and 173. These ions were shifted to m/z 169 and 187 in the spectrum presented in panel (F), due to the presence of an additional methyl group in the polar head. (H) MS3 spectrum of ion m/z 377 and structure and fragmentation pattern of C16 mLPA. (I) Part of the 1HNMR profile of the fraction collected at 17 min either without (lower inset) or with (upper inset) broadband decoupling on the 31P channel. Sections of the 13C-1H HSQC 41 spectrum showing the relevant cross peaks for the assignment of mLPA (right inset).

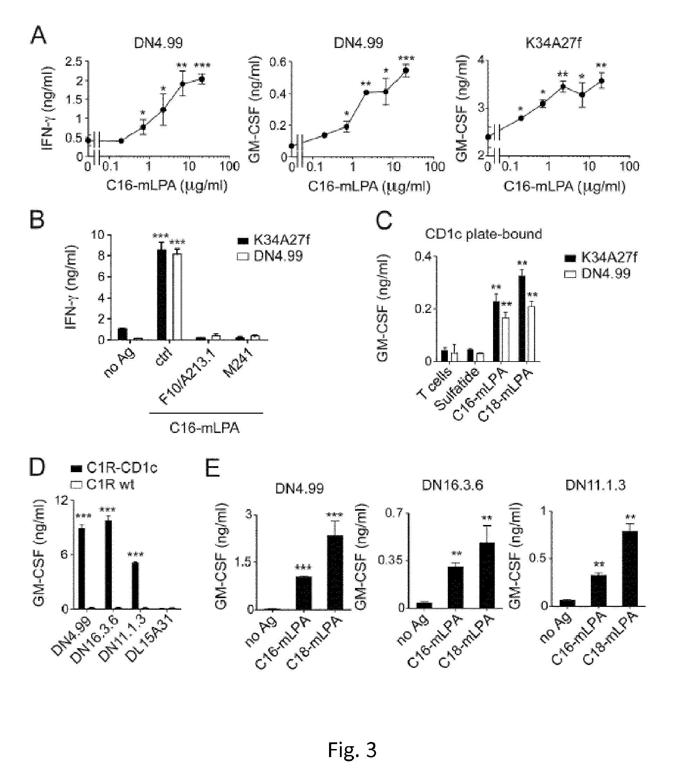

**Figure 3****. mLPA-specific T cells recognize synthetic mLPA analogs.** (A) T cell clones DN4.99 (left and middle panels) and K34A27f (right panel) were stimulated with synthetic C16 mLPA presented by purified primary B cells. (B) DN4.99 and K34A27f T cells were stimulated with purified primary B cells unloaded (no Ag) or loaded with C16 mLPA (10µg/ml) in the presence or not (ctrl) of two different anti-CD1c mAbs (F10/A213.1 or M241). (C) The same T cells were either cultured alone (T cells) or stimulated with plastic-bound sCD1c molecules loaded with control lipid (Sulfatide) or synthetic mLPA (C16 or C18). (D) Recognition of C1R-CD1c but not C1R wt by CD1c self-reactive T cell clones DN4.99, DN16.3.6 and DN11.1.3, each obtained from a different donor. The CD1c-restricted *M. tuberculosis-specific* DL15A31 T cell clone was used as a negative control for irrelevant specificity. (E) Activation of T cell clones DN4.99 (left panel), DN16.3.6 (middle panel) and
   DN11.1.3 (right panel) in the presence of purified primary B cells and synthetic C16 or C18 mLPA (or without antigen, no Ag). T-cell activation was evaluated by measurement of IFN-γ and/or GM-CSF release, expressed as mean ± SD. Results are representative of at least three independent experiments. *P < 0.05, **P < 0.01, ***P < 0.001, determined by two-tailed Student's t-test.

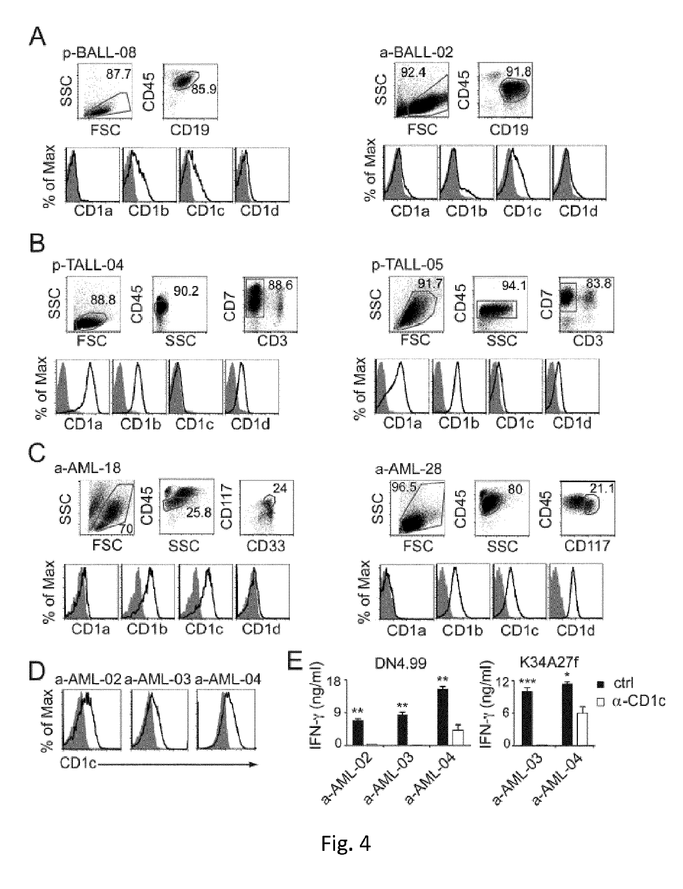

**Figure 4****. Primary leukemia blasts express CD1 molecules and stimulate mLPA specific T cells.** (A) CD1 expression by primary B-ALL blasts from a pediatric patient (p-BALL-08) and an adult patient (a-BALL-02). (B) CD1 expression by primary T-ALL blasts from two pediatric patients (p-TALL-04, p-TALL-05). (C) CD1 expression by primary AML blasts from two adult patients (a-AML-18, a42 AML-28). Gating strategies to identify the patient-specific leukemia phenotypes are shown in the upper panels. White histograms indicate specific staining of the gated populations with CD1-specific mAbs, grey histograms indicate the negative isotype-matched control staining (lower panels). (D) CD1c expression by primary AML blasts from three adult patients (a-AML-02, α-AML-03, α-AML-04). (E) Specific recognition of the same primary AML blasts by T cell clones DN4.99 (left panel) and K34A27f (right panel) at 1:10 E:T ratio, in the presence of irrelevant mAb (ctrl) or anti-CD1c blocking mAbs (α-CD1c). T cell activation was assessed by measuring IFN-γ release and was repeated twice with frozen aliquots of each leukemia sample.
   Data are expressed as mean ± SD. *P < 0.05, **P < 0.01, ***P < 0.001, determined by two-tailed Student's *t*-test.

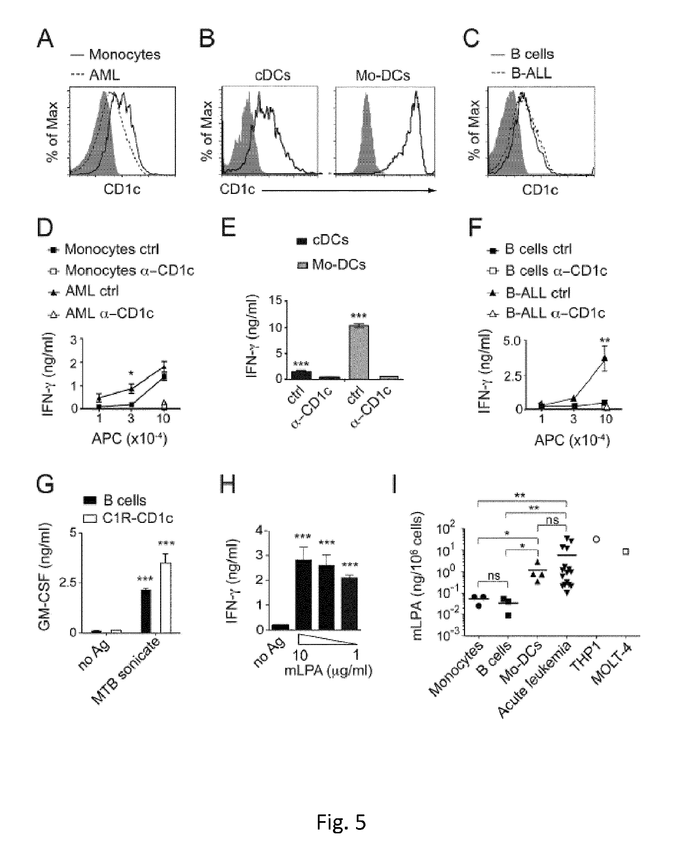

**Figure 5****. Differential recognition of malignant and healthy hematopoietic cells by mLPA-reactive T cells.** Expression of CD1c by (A) healthy donor-derived primary monocytes and primary AML blasts, (B) primary DCs purified from circulating mononuclear cells (cDCs) and monocyte-derived DCs (Mo-DCs), and (C) B cells from healthy donors and primary B-ALL blasts. (D-F) The same cell types were used to stimulate DN4.99 T cells in the presence of anti-CD1c mAbs (α-CD1c) or isotype-matched control mAbs (ctrl). IFN-γ release by T cells was measured and expressed as mean ± SD. (G) Normal B cells from efficiently presented *M*. *tuberculosis* lipid antigen (MTB sonicate) to CD1c-restricted *M. tuberculosis-specific* T cells (DL15A31). C1R-CD1c LCL were used as control. T cell activation was assessed by measuring release of GM-CSF, expressed as mean ± SD. (H) Normal B cells efficiently presented C16 mLPA to CD1c self-reactive T cells (DN4.99). T cell activation was evaluated by measuring release of IFN- y, expressed 43 as mean ± SD. Results are representative of three (A, B and C), three (D, E and F), three (G) and four (H) independent experiments. *P < 0.05, **P < 0.01, ***P <0.001, two-tailed Student's *t*-test. (I) C16 mLPA content in normal primary circulating monocytes (n=3) and B cells from healthy donors (n=3), *in vitro* differentiated monocyte-derived DCs (Mo-DCs, n=4), primary circulating B-ALL and AML blasts (Acute leukemia, n=5 and n=11, respectively), AML cell line THP1 and T-ALL cell line MOLT-4. In primary acute leukemia samples the percentage of blasts ranged between 75% and 97%. The mLPA concentration value of each sample represents the mean value of two to three independent determinations performed with the same sample. Horizontal bars represent the mean values of the primary samples. *P < 0.05, **P < 0.01, ns not statistically different, two tailed Student's *t-*test.

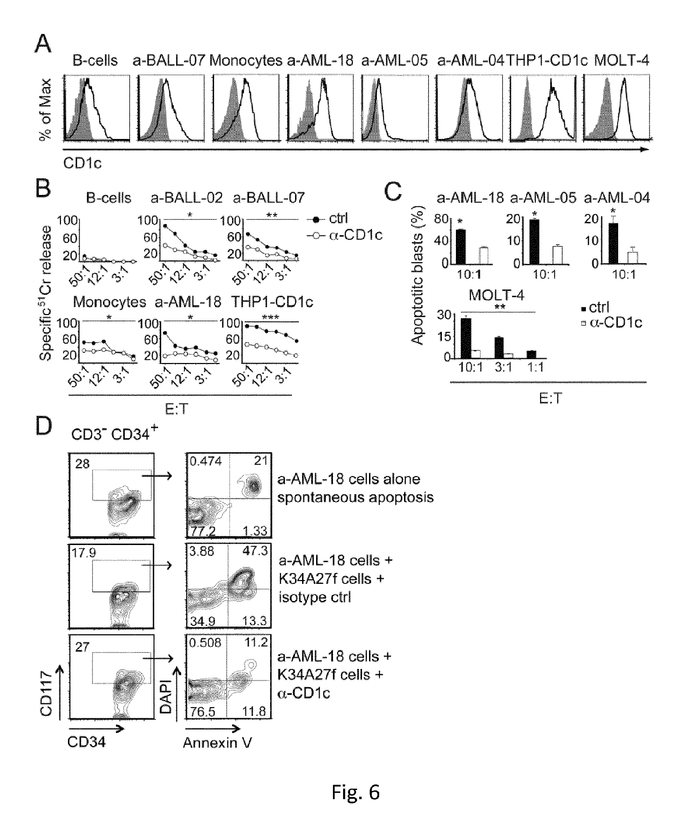

**Figure 6****. mLPA-specific T cells kill CD1c+ acute leukemia cells.** (A) CD1c expression by the indicated primary normal monocytes, primary AML blasts and leukemia cell lines. (B) Killing of 51 Cr-labeled primary AML and B-ALL blasts and normal purified monocytes and B cells. Cell targets were cultured with mLPA specific T cells (K34A27f) at increasing E:T ratio in the presence of isotype control (ctrl) or CD1c blocking mAbs (α-CD1c). 51Cr-labeled THP1-CD1c cells were used as positive control. Data are representative of two independent experiments. Two tailed paired Student's *t* test was used to compare the curves. (C) The indicated cell targets were cultured with mLPA-specific T cells (K34A27f) at the indicated E:T ratios in the presence of isotype control (ctrl) or CD1c blocking mAbs (α-CD1c). The percentages of apoptotic blasts were determined by flow cytometry using annexin V and DAPI staining. Columns indicate mean ± SD and are representative of two and three independent experiments with primary AML blasts and leukemia cell lines, 44 respectively. *P < 0.05, **P < 0.01, ***P < 0.001, determined two-tailed Student's *t* test.
   (D) FACS analysis of apoptotic primary AML blasts (a-AML-18) upon co-culture with mLPA-specific T cells (K34A27f) in the presence of isotype control mAbs (IgG1 isotype ctrl, middle panels) or anti-CD1c mAbs (〈-CD1c, lower panels). Spontaneous apoptosis of AML blasts in absence of T cells is also shown (upper panels). Leukemic cells responsible for the Minimal Residual Disease in treated patients were identified by co-expression of CD34 and CD 117 according to their specific LAIP (left panels). Apoptotic cells were defined by combined staining with DAPI and Annexin V (right panels). Data are representative of two independent experiments.

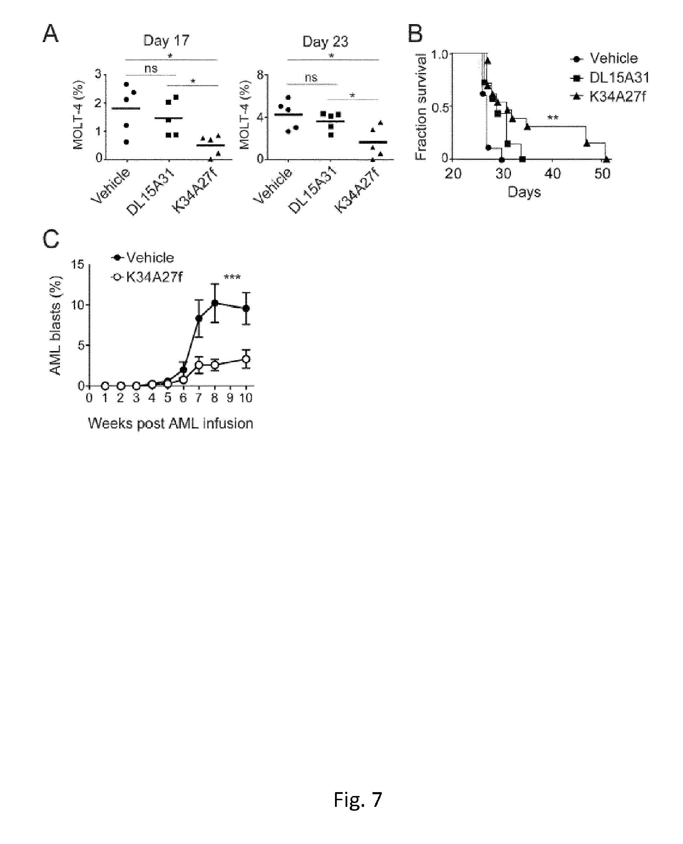

**Figure 7****. mLPA-specific T cells are promising tools for T cell adoptive immunotherapy of CD1c+ acute leukemia.** (A) Percentage of circulating MOLT-4 cells at different time points in mice inoculated with CD1c-restricted T cell clones specific for mLPA (K34A27f) or *M. tuberculosis* (DL15A31) or with saline only (Vehicle). *P < 0.05, ns, not significant, two-tailed Student's *t*-test. (B) Survival of mice that received either T cells or vehicle alone. Data show cumulative results from three independent experiments including 5 mice per group. Mice that received mLPA-specific T cells showed increased survival (**P < 0.01, Mantel-Cox test). (C) Progression of human primary CD1c+ AML blasts in NSG mice that received
   mLPA-specific K34A27f T cells (n=5) or Vehicle only (n=5). The percentage of AML blasts was monitored by flow cytometry in PBMCs at the indicated times. Bars indicate mean ± SD. Results are representative of two independent experiments. ***P < 0.001, non-parametric Student's *t*-test.

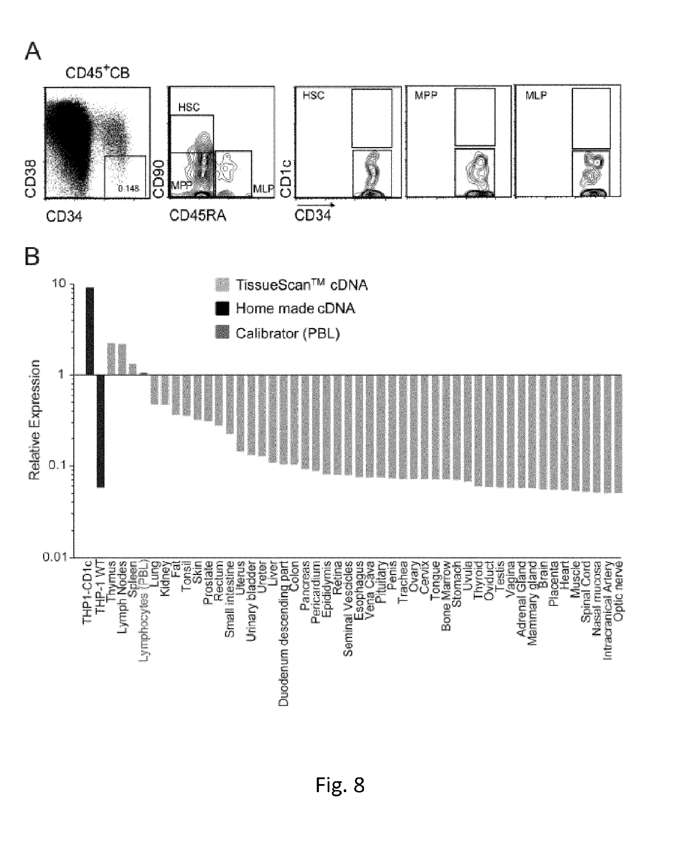

**Figure 8****. CD1c expression pattern in human hematopoietic precursors and healthy organs.** (A) Normal hematopoietic precursors lack CD1c expression. Cord blood cells from healthy donors were stained with the indicated mAbs (CD34-FITC, anti-CD45-APCCy7, CD38-PECy7, CD45RA-PB, CD90-APC, anti-CD1c-PE). The boxes represent the gates used to identify hematopoietic stem cells (HSC), multipotent progenitors (MPP), multilymphoid progenitors (MLP), according to (Doulatov et al., 2012). Representative dot plots from at least three independent experiments are shown. (B) CD1c mRNA expression in human tissues. RT-qPCR for CD1c expression was performed on cDNAs from human tissues contained in the TissueScanTM Real-Time Human Major Tissue Panels II, according to manufacturer instructions. The inventors added two custom cDNA, produced by us from THP1-CD1c and 46 THP1 WT cell lines, as positive and negative controls for CD1c expression. The data obtained were normalized against a calibrator signal, provided by the level of CD1c cDNA detection in PBMCs. CD1c expression was detected in lymph nodes, spleen and thymus, hereas was almost undetectable in all the other tissues.

### DETAILED DESCRIPTION OF THE INVENTION

### METHODS

### Cells

The following human cell lines were obtained from American Type Culture Collection: C1R (Epstein Barr Virus-transformed B lymphoblastoid), THP1 (myelomonocytc leukemia), MOLT-4 and Jurkat (T-ALL), CCRF-SB (B-ALL), and P3HR1 (Burkitt's lymphoma). THP1-CD1c were generated by transfection of human CD1c gene construct as previously described (Manolova et al., 2003). Primers used for PCR amplification were: forward *Xho*I-5'CTCGAGGACATGCTGTTTCTGCAGTTTC (SEQ ID No. 1) and reverse *Not*I-5'GCGGCCGCTCACAGGATGTCCTGATATGAGC (SEQ ID No. 2). C1R-CD1c cells were provided by S. Porcelli (Albert Einstein College, New York). Bone marrow and peripheral blood samples containing primary leukemia blasts were obtained after written informed consent from adult patients, or from the parents of pediatric patients, in accordance with the Declaration of Helsinki. The study was approved by the Institutional Review Boards of San Raffaele Scientific Institute, Milano, and of Fondazione IRCCS Policlinico San Matteo, Pavia. Primary AML, T-ALL and B-ALL leukemia samples were provided by Unita' Trapianto Midollo Osseo (San Raffaele, Milano), Northern Italy Leukemia Group (Bergamo) and A. Wodnar-Filipowicz (University of Basel). T cell clones were generated and maintained in culture as previously described (de Lalla et al., 2011; Shamshiev et al., 1999). Normal monocytes and B cells were purified (>90% purity) from PBMCs obtained from healthy volunteers using anti-CD14 and anti-CD19 immunomagnetic beads, respectively, according to the manufacturers' instructions (Miltenyi Biotec). cDCs were enriched from PBMCs using Human DC Enrichment beads (Invitrogen) and the unbound fraction was sorted according to the expression of CD64 and CD11c. The resulting CD64⁺CD11c⁺ cDCs (>95% purity) uniformly expressed CD1c. Mo-DCs, were differentiated *in vitro* from purified CD14+ monocytes in presence of GM-CSF and IL-4 as described (Shamshiev et al., 1999).

### Flow cytometry

Expression was determined using mAbs anti-CD1a-PE (clone HI149 BD Pharmingen), anti-CD1b-PE (clone 0249, Santa Cruz Biotechnology), anti-CD1c-PE (clone L161, Santa Cruz Biotechnology), and anti-CD1d-PE (clone 42.1, BD Pharmingen). Leukemia blasts were identified using anti-CD19-FITC, or CD19-PerCPCy5.5 (both clone HIB 19), anti-CD14-FITC (clone HCD14) (all from BioLegend), anti-CD3-FITC (clone UCHT1), anti-CD45-APC (clone HI30), anti-CD117-PerCpCy5.5 (clone 2B8) (all from BD Pharmingen), anti-CD7-PC5 (clone 8H8.1), anti-CD10-PC5 (clone ALB1), anti-CD33-FITC or CD33-PC5, anti-CD33-APC (all clone D3HL60-251), anti-CD34-FITC or CD34-PC5, or anti-CD34-APC (all clone 581), anti-CD45-PC7 (clone J33) (all from Coulter). cDCs were sorted using anti-CD64 FITC (clone 22, Coulter) and anti-CD11c APC (clone B-ly6 BD Pharmingen).

Samples were acquired on CyAn ADP (DakoCytomation) or FACS Canto (Becton Dickinson) flow cytometers. Cell sorting experiments were performed using a MoFlo sorter (Beckman-Coulter). Dead cells were excluded on the basis of propidium iodide and DAPI staining. All data were analyzed using FlowJo software (TreeStar). Relative Fluorescent Intensity (RFI) of CD1c expression on leukemia cells was calculated dividing the mean CD1c fluorescence intensity by the mean fluorescence intensity of the corresponding isotype control mAb.

### T cell activation assays

T cell activation assays were performed by co-culturing the indicated numbers of target cells (5x104/well unless otherwise indicated) with T cells (5x104/well) in 200µl volume in duplicate or triplicate cultures. In some experiments, mouse anti-CD1c (L161, Abcam) or mouse IgG1 isotype control mAbs were added and incubated for 30 min prior to the addition of T cells. Supernatants were collected after 24-48 hours and cytokines released by T cells (GM-CSF and/or IFN-γ) were measured by ELISA. In assays using fractionated lipids, THP1-CD1c cells (5x104/well) were fixed with 0.05% glutaraldehyde for 20 s and used as APCs. Fold change in cytokine release was calculated using the following formula: GM-CSF release with lipid pulsed APC / GM-CSF release with un-pulsed APC. In some experiments, normal B cells purified from PBMCs (5x104/well) were used as APCs without prior fixation. APCs were pulsed with lipids for 4 hours at the indicated dilutions before addition of T cells.

sCD1c-plate-bound assays were performed using MaxiSorp plates (Nunc) coated with anti-BirA mAb (Nowbakht et al., 2005) to capture sCD1c and were then incubated with lipids for 6 hours. The wells were extensively washed before addition of T cells.

Each stimulation was performed in triplicate. Killing assays were performed using target primary blasts or established leukemia cell lines (2x104 cells/ml) incubated either alone or with T cells at different effector: target (E:T) ratios for 24 hours, in the presence or absence of anti-CD1c mAb (L161). Killing of 51Cr-labelled targets were performed as described (Montagna et al., 2006). The mean spontaneous release ranged between 15% and 22% of total release. In the killing assays performed by flow cytometry, the target cells were stained with PE-Annexin V (BD Pharmingen) and 4',6-diamidino-2-phenylindole DAPI (Sigma), according to published protocols (Jedema et al., 2004). Leukemia blasts were identified by their leukemia specific associated immune phenotypes (LAIP) and then assessed for apoptosis as follows: Annexin V+ DAPI+ = advanced apoptosis, and Annexin V- DAPI+ = necrosis. The percentage of apoptotic + necrotic cells in the absence of T cells (spontaneous apoptosis) was subtracted from the percentage of apoptotic + necrotic cells in the presence of T cells (induced apoptosis) to obtain the proportion of blasts killed by T cells.

### Production of purified sCD1c

Recombinant soluble human CD1c tagged with BirA peptide (sCD1c), was obtained and purified using techniques similar to those previously described (Garcia-Alles et al., 2006). The following primers were used to PCR amplify the extracellular domain of human CD1c: forward *Xho*I 5'- CTCGAGGACATGCTGTTTCTGCAGTTTC (SEQ ID No. 3) and reverse *Cla*I 5'-CCATCGATGGGGTTTCTCCAGTAGAGGA (SEQ ID No. 4). sCD1c was purified from J558-transfected cell culture supernatants by affinity chromatography using anti-CD1c mAb (10C3, BD Pharmingen).

### Lipid extraction and analysis

Total lipids were extracted from a dry pellet of 109 THP1 cells with water:methanol:chloroform (1:1:2 vol:vol:vol). The chloroform layer was collected, centrifuged, dried and dissolved in chloroform:methanol (9:1 vol:vol). Highly polar lipids were extracted from the remaining aqueous phase using butanol (1 vol added), then centrifuged, dried and re-dissolved in chloroform:methanol (9:1 vol:vol). Both organic and aqueous extractions were separately loaded on an amino-propyl Sep-Pak cartridge (Waters Corporation, Massachusetts, USA) and lipids were fractionated into 10 fractions as previously described (Facciotti et al., 2012). No structural modifications were observed in a mixture of standard lipids subjected to the same experimental procedures, thus excluding qualitative effects of the solvents and the amino propyl cartridges on the eluted lipids. Sub-fractionation of fraction G was performed by HPLC using a reverse phase C18 polar end-capped column (3µm particle size, 3mm ID, 125mm length, Macherey-Nagel) as previously described (Facciotti et al., 2012). LC-MS-MS was performed using a Rheos Allegro pump (Flux Instruments, Switzerland) and HTC PAL injector with a 20µl loop (CTC Analytics 26 AG, Switzerland). MSn were acquired using an LXQ ion trap mass spectrometer equipped with a heated ESI source (Thermo, San Jose, CA, USA) controlled by Xcalibur software (Thermo Finnigan). Source conditions were: spray voltage, 5kV; capillary voltage, 12V; capillary temperature, 265°C; tube lens offset voltage, 100V; sheath gas flow rate, 15 units. Helium was used as the damping gas. CID spectra were acquired on an API 4000 LC/MS/MS system with triple quadrupole analyzer (Applied Biosystems/ MDS Sciex, Foster city, CA, USA). Exact mass measurements were carried out with LTQ MS (Thermo, San Jose, CA, USA). All lipid standards were purchased from Avanti polar (Alabama, USA).

To quantify the amounts of C16 mLPA contained in leukemic and healthy cells, polar lipids were extracted with a one-step methanol-based method (Zhao and Xu, 2010).

Briefly, dry cell pellets were frozen in liquid nitrogen (5 min), then kept on ice for 3 min and finally brought at room temperature in water for 2 min. Samples were vortexed (30 s), added of 50 µl of water, vortexed for additional 30 s, added of 450 µl of methanol, vortexed again (30 s) and finally sonicated for 3 min. After centrifugation (10 min, 10,000g), the upper phase was collected and dried under fume hood. Dry lipid extracts were resuspended in 30µl of methanol and 5µl were injected into the Ultra Performance Liquid Chromatography - tandem Mass Spectrometer (UPLC-MS-MS) for analysis. UPLC was carried out on a Waters ACQUITY UPLC system (Waters Corp., Milford, MA) using a 2.1 mm × 100mm x1.7 µm ACQUITY UPLC BEH C18 column with a flow rate of 0.20 ml/min. The column temperature was held at 30°C. The solvent system applied was: mobile phase A (2mM ammonium formate in Milli-Q water) and mobile phase B (acetonitrile, HPLC grade) with a gradient starting at 70%A-30% B and ramping to 100% B over 10 min. Mass spectrometry detection was performed using a Waters Xevo TQS Tandem MS 27 detector (Waters Corp., Milford, MA) equipped with an electrospray ionization source (ESI) operating in negative ionization and multiple reaction monitoring (MRM) mode. The desolvation gas flow rate was set to 800 1/h at a temperature of 350°C. The capillary voltage was set to 2500 V. The precursor to product ion transitions (quantification and target ions), dwell times, cone voltage, and collision energy for C16 mLPA and the internal standard C 17 mLPA are listed in Table S3.

**Table S3. C16 and C 17 mLPA MRM transitions**

| Compound | Transition | Cone voltage | Collisional | Dwell |
|---|---|---|---|---|
| C16 mLPA | 408.96>110.84^{a} | 20 | 25 | 0.025 |
| | 408.96>376.97 | 20 | 35 | 0.025 |
| C17 mLPA | 423.06>110.89^{a} | 20 | 25 | 0.025 |
| | 423.06>391.02 | 20 | 35 | 0.025 |

| | | | | |
|---|---|---|---|---|
| ^{a}Quantification ion. | | | | |

Data were acquired and processed with Waters MassLynx software v.4.1, using peak area ratio for quantification. C16 and C17 mLPA molecules were synthesized in house.

### NMR

mLPA spectra were recorded on a Bruker Avance III spectrometer operating at 14.1 Tesla and 298K. The spectrometer was equipped with a triple resonance (1H, 13C, 31P) 1.7mm micro probe-head with a self-shielded z-gradient coil. All mLPA experiments were performed in sodium formate-buffered deuterated methanol (99.96 % D, Cambridge Isotopes Laboratories, Andover, MA, USA). All synthetic samples were prepared in deuterated solvents (>99.8 % D, Cambridge Isotope Laboratories, Burgdorf, Switzerland). The NMR experiments conducted on synthetic samples were performed using a Bruker DRX-600 MHz NMR spectrometer (equipped with a selfshielded z-axis pulsed field gradient dual-channel broadband inverse 5mm probehead). Chemical shifts were referenced to residual solvent peaks. mLPA was dissolved in 30µl solvent, syringe-transferred to a 1.7mm diameter NMR sample tube and then sealed. The two-dimensional experiments were recorded with 2048 data points in the direct dimension, and increments in the indirect dimension numbering 1024 (COSY and HSQC) or 256 (31P-HMBC), resulting in acquisition times of 285ms (F2) and 142ms (F1) for the COSY, 142ms and 20.5ms for the HSQC, and 142ms and 10.5ms for the 31P-HMBC. The COSY was performed with 8 scans per increment leading to a total experiment time of 3 hours, 21 min, for the HSQC these parameters were 144 scans and 64 hours, 17 min and for the 31P-HMBC 8 scans and 59 min. A 31P decoupling 180° pulse was applied during the 13C evolution period of the sensitivity-enhanced Echo-Antiecho HSQC experiment. The long-range delay in the 31P-HMBC was set to 62.5ms. All other NMR investigations were routine experiments.

### Human leukemia xenograft model

### The inventors generated a human leukemia xenograft model in immune compromised mice

according to published protocols (Bondanza et al., 2006; Provasi et al., 2012). TMβ-1 antimouse IL-2Rβ blocking mAb (clone TM-1, provided by K. Tanaka) was injected *i.p.* 24 hours before the start of the experiment in 6-8 weeks old female NOD/scid mice (Charles River) to neutralize residual NK cell activity. On day 0, mice were conditioned by 175 cGy sub-lethal irradiation and then immediately infused *i.v*. with 106 MOLT-4 cells engineered to express EGFP via lentivirus transfer. After 48 hours, the mice were divided into three groups (5 animals/group), that received either vehicle alone, mLPA-specific CD1c-restricted T cells (K34A27f), o*r M. tuberculosis-specific* CD1c-restricted T cells (DL15A31) (10⁷ cells/mouse). The proliferation of human leukemia cells in blood was monitored weekly by flow cytometry. MOLT-4 cells were identified as human-CD45/EGFP double positive cells. Moribund mice were euthanized for ethical reasons. For studies with primary human leukemia blasts, AML blasts were purified from the blood of an AML patient and injected *i.v.*

(8x10⁶/animal) into 14 NOD/scid/common γ chain-/- (NSG) mice. Two days later, 1.5x10⁷ K34A27f T cells were injected into half of the mice and leukemia progression was monitored weekly by flow cytometry of PBMCs. The area under the curve describing the kinetic of AML expansion in peripheral blood was calculated for each mouse infused with K34A27f cells or with vehicle only and values derived from the two groups of animals compared. Animal studies were approved by the Institutional Animal Care and Use Committee of the San Raffaele Scientific Institute (IACUC n. 370).

### Statistical analyses

Cytokine assay data were analyzed using a two-tailed Student's *t*-test. Data describing the presence of leukemia cells in injected mice were analyzed with two-tailed nonparametric Student's *t*-test. Animal survival data were analyzed using log-rank (Mantel-Cox) test.

### Clinical characteristics of pediatric and adults patients

**Table S1: clinical characteristics of pediatric patients**

| Pediatric AML | | | | |
|---|---|---|---|---|
| Patient | Gender/Age | Malignancy^{a} | Risk^{b} | Blast CD1 expression |
| p-AML-01 | M/15 | AML | H | CD1b-CD1c |
| p-AML-02 | M/15 | sAML MD | H | CD1b-CD1c |
| p-AML-03 | F/12 | AML | H | CD1b-CD1c |
| p-AML-04 | M/18 | AML | L | CD1b-CD1c |
| p-AML-05 | F/8 | AML | L | Negative |
| p-AML-06 | F/18 | AML | H | Negative |
| p-AML-07 | F/2 | sAML MD | H | Negative |
| p-AML-08 | F/1 | AML | H | Negative |
| p-AML-09 | F/7 | AML | H | Negative |
| | | | | |

| Pediatric B-ALL | | | | |
|---|---|---|---|---|
| Patient | Gender/Age | Malignancy | Risk | Blast CD1 profile |
| p-BALL-01 | M/3 | B-ALL | I | CD1c-CD1d |
| p-BALL-02 | M/4 | B-ALL | H | CD1b-CD1c |
| p-BALL-03 | M/5 | B-ALL | I | Negative |
| p-BALL-04 | F/6 | B-ALL | H | Negative |
| p-BALL-05 | F/2 | B-ALL | S | Negative |
| p-BALL-06 | F/13 | B-ALL | S | Negative |
| p-BALL-07 | F/2 | B-ALL | I | Negative |
| p-BALL-08 | M/8 | B-ALL | H | CD1b-CD1c |
| p-BALL-09 | M/7 | B-ALL | H | Negative |
| p-BALL-10 | F/8 | B-ALL | S | Negative |
| p-BALL-11 | F/9 | B-ALL | S | Negative |
| p-BALL-12 | M/16 | B-ALL | H | Negative |
| p-BALL-13 | F/6 | B-ALL | S | Negative |
| p-BALL-14 | M/- | B-ALL | I | CD1b-CD1c |
| p-BALL-15 | M/7 | B-ALL | S | Negative |
| p-BALL-16 | F/4 | B-ALL | H | CD1c |
| p-BALL-17 | F/4 | B-ALL | I | Negative |
| p-BALL-18 | F/3 | B-ALL | I | Negative |
| p-BALL-19 | M/3 | B-ALL | S | Negative |
| p-BALL-20 | F/- | B-ALL | n.e. | CD1a-CD1b |
| p-BALL-21 | M/9 | B-ALL | S | Negative |
| p-BALL-22 | F/13 | B-ALL | I | Negative |
| p-BALL-23 | F/9 | B-ALL | I | CD1b-CD1c |
| | | | | |

| Pediatric T-ALL | | | | |
|---|---|---|---|---|
| Patient | Gender/Age | Malignancy | Risk | Blast CD1 profile |
| p-TALL-01 | M/5 | T-ALL | H | CD1a-CD1b |
| p-TALL-02 | M/14 | T-ALL | S | CD1a-CD1b-CD1d |
| p-TALL-03 | M/7 | T-ALL | H | CD1a-CD1b |
| p-TALL-04 | M/7 | T-ALL | S | CD1a-CD1b-CD1d |
| p-TALL-05 | M/7 | T-ALL | H | CD1a-CD1b-CD1c-CD1d |
| p-TALL-06 | M/3 | T-ALL | S | CD1a-CD1b-CD1d |
| p-TALL-07 | F/8 | T-ALL | H | Negative |
| p-TALL-08 | F/7 | T-ALL | H | Negative |

| | | | | |
|---|---|---|---|---|
| ^{a}AML, Acute Myeloid Leukemia; sAML MD, Secondary Acute Myeloid Leukemia from Myelodisplastic Syndrome; B-ALL, B Acute Lymphoblastic Leukemia; T-ALL, T Acute Lymphoblastic Leukemia; bDisease Risk: H, high risk; S, standard risk; I, intermediate risk; L, low risk; n.e., not evaluated. | | | | |

**Table S2: Clinical characteristics of adults patients**

| Adult AML | | | | |
|---|---|---|---|---|
| Patient | Gender/Age | Malignancy^{a} | Risk^{b} | Blast CD1 profile |
| a-AML-01 | M/39 | sAML | A | Negative |
| a-AML-02 | M/56 | AML | A | CD1b-CD1c |
| a-AML-03 | F/29 | AML | A | CD1b-CD1c |
| a-AML-04 | F/54 | AML | F | CD1b-CD1c-CD1d |
| a-AML-05 | F/64 | sAML MD | A | CD1b-CD1c-CD1d |
| a-AML-06 | F/38 | AML | I | Negative |
| a-AML-07 | F/59 | AML | A | CD1b-CD1c-CD1d |
| a-AML-08 | F/64 | AML | I | CD1b-CD1c |
| a-AML-09 | M/21 | sAML | A | CD1b-CD1c |
| a-AML-10 | F/28 | sAML MD | A | CD1b-CD1c |
| a-AML-11 | F/53 | AML | A | Negative |
| a-AML-12 | F/53 | AML | A | CD1b-CD1c |
| a-AML-13 | F/56 | AML | A | CD1b-CD1c-CD1d |
| a-AML-14 | M/74 | AML | I | Negative |
| a-AML-15 | M/64 | AML | A | Negative |
| a-AML-16 | M/77 | AML | A | CD1b-CD1c |
| a-AML-17 | F/62 | AML | I | Negative |
| a-AML-18 | M/45 | AML | A | CD1b-CD1c |
| a-AML-19 | F/59 | AML | A | None |
| a-AML-20 | M/60 | sAML MD | A | CD1b-CD1c |
| a-AML-21 | M/69 | AML | F | CD1a |
| a-AML-22 | F/58 | sAML MD | A | Negative |
| a-AML-23 | F/50 | AML | F | CD1d |
| a-AML-24 | M/52 | AML | A | CD1d |
| a-AML-25 | F/52 | AML | A | Negative |
| a-AML-26 | F/84 | AML | A | CD1b |
| a-AML-27 | F/70 | AML | A | Negative |
| a-AML-28 | F/39 | AML | A | CD1b-CD1c-CD1d |
| a-AML-29 | F/39 | AML | A | CD1b-CD1c-CD1d |
| a-AML-30 | M/57 | AML | F | CD1b-CD1c |
| a-AML-31 | M/42 | AML | A | Negative |
| a-AML-32 | F/67 | AML | A | CD1b-CD1c |
| a-AML-33 | F/61 | sAML | A | Negative |
| | | | | |

| Adult B-ALL | | | | |
|---|---|---|---|---|
| Patient | Gender/Age | Malignancy | Risk | Blast CD1 profile |
| a-BALL-01 | M/61 | B-ALL | VH | Negative |
| a-BALL-02 | M/78 | B-ALL | VH | CD1c |
| a-BALL-03 | F/47 | B-ALL | VH | CDlc |
| a-BALL-04 | M/22 | B-ALL | n.e. | Negative |
| a-BALL-05 | M/42 | B-ALL | VH | CD1c, CD1d |
| a-BALL-06 | F/38 | B-ALL | H | CD1c, CD1d |
| a-BALL-07 | F/78 | B-ALL | VH | CD1c |

| | | | | |
|---|---|---|---|---|
| ^{a}AML, Acute Myeloid Leukemia; sAML MD, Secondary Acute Myeloid Leukemia from Myelodisplastic Syndrome; B-ALL, B Acute Lymphoblastic Leukemia; ^{b}Disease Risk for AML: A, Adverse, I, Intermediate, F, Favorable; ^{b}Disease Risk for B-ALL: VH, Very High Risk; n.e., not evaluated. | | | | |

### RESULTS

### Identification of CD1c-presented antigenic lipids in leukemia cells

Auto-reactive T cells restricted to CD1c are abundant in the peripheral blood of healthy donors (de Lalla et al., 2011) and are activated in the absence of exogenous antigens, suggesting that they target cells expressing endogenous molecules presented by CD1c. Since CD1c is exclusively expressed on hematopoietic cells, we initially evaluated the capacity of different tumor cell lines of hematopoietic origin to activate CD1c auto-reactive T lymphocytes. Two CD1c selfreactive T cell clones isolated from separate donors were stimulated with CD1c gene-transfected C1R cells (C1R-CD1c, representative of Epstein Barr virus transformed lymphoblastoid cells), and THP1 cells, (THP1-CD1c, representative of acute myeloid leukemia, AML), and with four other cell lines which naturally expressed CD1c: CCRF-SB (a B-acute lymphoblastic leukemia, B-ALL,), MOLT-4 and Jurkat (established from T-acute lymphoblastic leukaemias, T-ALL), P3HR1 (a Burkitt's lymphoma) (Fig. 1 A). All six tumor cell lines induced T cell production of GM-CSF (Fig. 1 B) and IFN-γ (data not shown) in a CD1c-dependent manner, as indicated by full inhibition of target recognition with blocking anti-CD1c monoclonal antibodies (mAbs) (Fig. 1 C). Hence, CD1c self-reactive T cells were stimulated by a broad range of hematological malignancies, raising the question as to which type of common self-lipid antigens stimulate these cells.

To identify the common lipid antigens, the inventors used combined biochemical approaches in conjunction with T cell activation assays. Total lipids were extracted from the THP1 cell line, which most efficiently stimulated both CD1c-restricted T cell clones (Fig. 1 C). Separation of the total lipid mixture based on polarity and charge (Facciotti et al., 2012) revealed only one lipid fraction (G) that was capable of supporting the dose-dependent activation of self-reactive T cells by fixed CD1c+APCs (Fig. 1 D). Fraction G also stimulated T cells when presented by plastic-bound recombinant soluble CD1c (sCD1c), whereas anti-CD1c mAb inhibited T cell activation in these assays (Fig. 1E left and middle panels). Control antigen sulfatide (Shamshiev et al., 2002) failed to stimulate T cells in these experiments, and an irrelevant CD1c-restricted T cell clone (specific for *M*. *tuberculosis* lipids) did not respond to fraction G, thus confirming the antigen specificity of the response (Fig. 1E right panel).

HPLC purification of the lipids in fraction G (Fig. 2 A) indicated that the lipids eluted at min 17, 18 and 19.5 were capable of stimulating CD1c self-reactive T cells (Fig. 2 B). Liquid chromatography-tandem mass spectrometry (LC-MS-MS) was then used to re-analyze the lipids obtained at min 17 to better determine the structure of the tumor-associated lipid antigens. A single lipid was eluted at min 17 (Fig. 2 C), and this compound exhibited a mass/charge (m/z) value of 409 in negative ion mode (Fig. 2 D). Since this mass did not match any known lipids, a detailed biochemical investigation was performed in order to identify the novel structure.

Tandem mass spectrometry (MS2) fragmentation of the novel compound yielded an ion of m/z 377 (Fig. 2 E), corresponding to the loss of methanol (32Da). The detection of an additional ion of m/z 167 in the low mass region of the MS2 spectra indicated the presence of a phosphate moiety (signature ion m/z 153) (Pulfer and Murphy, 2003), and an associated methyl group (m/z 14). Based on these data, we assigned a methylated phosphate head to the structure of the antigenic lipid.

The inventors next used positive ion mode analyses to confirm the candidate structure proposed by the negative ion mode data. The protonated ion of the novel compound (m/z 411) generated a fragment of m/z 299 and associated loss of 112Da in mass, 8 corresponding to the loss of a methyl hydrogen phosphate moiety (Fig. 2 F). Ions with m/z of 169 and 187 were detected in the low mass region (Fig. 2 F), consistent with the presence of a 14Da methylated head group bound to phospholipids of 155 or 173Da (Fig. 2 G). Additional experiments were then performed using a triplequadrupole analyzer to confirm the presence of a phospho-methyl group within the novel lipid antigen. Upon collision-induced dissociation, the deprotonated ion of m/z 409 generated ions of m/z 377 and 111 (data not shown). An excess mass of 14 Da again pointed to the presence of a methylated phosphate group (m/z 97 + 14).

To elucidate the structure of the hydrophobic chain of the novel lipid antigen, the inventors used triple-stage mass spectrometry (MS3) in negative mode with an ion-trap instrument. Fragmentation of ion m/z 377 generated a further ion of m/z 239 (Fig. 2 H) indicative of an ether-bonded C16 alkyl or alkenyl chain. However, prominent peaks indicative of alkenyl chains (Han and Gross, 1996; Hsu and Turk, 2007) were absent in the earlier MS2 analysis, thereby excluding this structural feature. The presence of a reproducible, low intensity hexadecyl-carbocation at m/z 225 in the MS2 spectra of the protonated molecule further supported the presence of an alkyl chain (Fig. 2 F). Together, the MS data defined the lipid antigen eluted at min 17 as a lyso-glycero-methyl hydrogen phosphate with an ether-bonded C16 alkyl chain (1-O-hexadecyl-sn-glycero-3-phosphoric acid methyl ester, or methyl-lysophosphatidic acid; mLPA) (Fig. 2 H). High-resolution mass spectrometry analysis (HRMS) supported this conclusion by revealing a protonated molecular mass of 411.2872 Da, compatible with the theoretical mass of the proposed molecule to within 0.5ppm error (data not shown).

In order to confirm the proposed structure of the novel lipid, full assignment and structural elucidation of the compound was undertaken using one- and two dimensional NMR spectroscopy. A prominent signal was detected at 3.58 ppm in the proton NMR spectrum, corresponding to the methoxy group on the phosphate portion of the novel lipid. The resonance reading for this phosphate moiety appeared as doublet due to scalar coupling to the 31P nucleus (3JHP=10.9 Hz), but the doublet was resolved by decoupling (Fig. 2 I). Correlation spectroscopy (COSY) unraveled the complex pattern of the five glycerol protons (data not shown). The inventors then applied a two-dimensional 13C-1H heteronuclear single quantum coherence spectroscopy correlation (HSQC) to identify and assign all carbons and protons within the molecule (Fig. 2 I right inset), and validated this assignment using a 31P-1H heteronuclear multiple bond correlation (HMBC) to demonstrate the interaction of the phosphorus atom with the methoxy group and the H3 protons (data not shown).

In support of these data, the inventors prepared synthetic mLPA analogs that showed identical chemical shifts in the 1H, 13C, and 31P spectra, and recorded spectra from 1H-1H nuclear Overhauser effect spectroscopy (NOESY) and long-range 13C-1H HMBC that were consistent with the postulated structure (data not shown).

A similar structure was also identified in the second active sub-fraction of THP1 lipid extract collected at min 18 (Fig. 2 B). Analysis of this molecule revealed a second lyso-glycero-methyl hydrogen phosphate that contained an ether-bonded C18-alkyl chain (data not shown), which was longer than the C16 alkyl chain present in the compound eluted at min 17. Indeed, the inventors characterized the stimulatory compound collected at min 18 as a C18 mLPA (data not shown).

The inventors next used synthetic analogs of the naturally occurring molecules (identical mass spectra and NMR behavior; data not shown) to test the antigenic potency of the novel lipids. The synthetic compounds very efficiently activated T cells when presented by CD1c+ APCs with potency ranging between 0.5 and 2 µg/ml (Fig. 3 A). Two anti-CD1c monoclonal antibodies (mAbs) completely inhibited the response (Fig. 3 B). The synthetic analogs also stimulated T cells when loaded onto plastic-bound sCD1c molecules, thus confirming the recognition of the CD1c-mLPA complexes (Fig. 3 C). The same T cells were very weakly stimulated by other more common phospholipids (LPA, LPC and LPE) only at higher doses, above 50 µg/ml, suggesting high specificity for mLPA (data not shown). Two additional CD1c self-reactive T cell clones isolated from a further two healthy donors (Fig. 3 D) and bearing different TCR α/β chains, were also capable of recognizing the synthetic analogs (Fig. 3 E), indicating that T cells specific for the novel lipids are present in healthy individuals. Collectively, these data identified the previously unknown self-lipid antigen mLPA as a potent agonist for CD1c-restricted human T cells.

### Primary leukemia blasts express CD1 molecules

Having identified a novel self-lipid antigen that was presented by CD1c in leukemia cell lines, the inventors next investigated the frequency of CD1c expression by leukemia blasts in patients. Peripheral blood and bone marrow samples obtained from AML, B-ALL and T-ALL patients (Table S1 and S2) were analyzed for the expression of different CD1 isoforms. Malignant cells were identified according to specific leukemia-associated cellular phenotypes (Fig. 4). All leukemia blasts expressed CD1 molecules, although at different levels in different patients (Table 1 and Fig. 4).

**Table 1. CD1 expression by primary leukemia blasts**

| **Pediatric patients** | | | | | |
|---|---|---|---|---|---|
| Malignancy¹ | Number | Patients with CD1-expressing blasts (%) | | | |
| | | CD1a | CD1c | CD1c | CD1d |
| AML | 9 | 0 | 45 | 45 | 0 |
| B-ALL | 23 | 4 | 22 | 26 | 4 |
| T-ALL | 8 | 75 | 75 | 12 | 50 |
| | | | | | |

| **Adult patients** | | | | | |
|---|---|---|---|---|---|
| Malignancy¹ | Number | Patients with CD1-expressing blasts (%) | | | |
| | | CD1a | CD1b | CD1c | CD1d |
| AML | 33 | 3 | 54 | 51 | 24 |
| B-ALL | 7 | 0 | 0 | 71 | 29 |

| | | | | | |
|---|---|---|---|---|---|
| ¹Data refer to circulating blasts. Similar CD1 expression frequencies were observed in bone marrow biopsy samples. | | | | | |

AML blasts from pediatric patients expressed CD1b (45% of patients) and CD1c (45%), but lacked CD1a and CD1d (Table 1). Pediatric B-ALL blasts expressed CD1b (22%), and CD1c (26%) but rarely expressed either CD1a (4%) or CD1d (4%) (Table 1 and Fig. 4 A). Pediatric T-ALL blasts expressed CD1a (75%), 11 CD1b (75%), and CD1d (50%), but displayed CD1c at much lower frequency (12%) (Table 1 and Fig. 4 B). In adult patients, AML blasts expressed CD1b (54%), CD1c (51%), and CD1d (24%) but little CD1a (3%) (Table 1 and Fig. 4 C). B-ALL blasts expressed CD1c (71%) and CD1d (29%), while CD1a and CD1b were undetectable (Table 1 and Fig. 4 A). CD1 molecules are thus frequently present on leukemia blasts.

The expression of CD1 molecules was not associated with disease risk or particular clinical, cytogenetic and molecular prognostic features (Table S1 and S2) (Dohner et al., 2010; Pui et al., 2011).

### Differential recognition of primary leukemia blasts and healthy hematopoietic cells by mLPA-specific T cells

Because CD1c is expressed by a large proportion of acute leukemia blasts, the inventors studied whether mLPA-specific T cells also recognize primary leukemia cells in a CD1c-dependent manner. Primary CD1c+ AML blasts freshly isolated from three different patients (Fig. 4 D) induced IFN-γ release by two mLPA-specific T cell clones and their recognition was blocked by anti-CD1c mAbs (Fig. 4 E).

The inventors next assessed whether mLPA-specific T cells recognized cancer as well as normal CD1c+ cells. Initially, the inventors examined the myeloid compartment. The selected AML blasts expressed lower levels of CD1c than normal circulating monocytes and dendritic cells (cDCs) (Fig. 5A and B), nevertheless they elicited higher levels of T cell activation than normal monocytes (Fig. 5 D), and similar to that of cDCs (Fig. 5 E), suggesting that AML blasts act as potent stimulatory cells. *In vitro* differentiated monocyte-derived dendritic cells (Mo-DCs) displayed high expression of CD1c (Fig. 5 B) and also exhibited strong T cell stimulatory activity (Fig. 5 E). The recognition of all four cell types was fully inhibited by anti-CD1c 12 mAbs (Fig. 5D and E).

Next the inventors determined CD1c-restricted T cell recognition of transformed and normal B cells. Primary CD1c+ B-ALL blasts and healthy B cells displayed comparable CD1c expression levels (Fig. 5 C), yet the leukemic cells induced markedly higher production of IFN-γ by mLPA-reactive T cell clones (Fig. 5 F).

Also in this case, anti-CD1c mAbs completely blocked the recognition of B-ALL (Fig. 5 F). Normal B cells were able to present exogenously loaded synthetic mLPA or bacterial-derived lipids to specific CD1c-restricted T cell clones (Fig. 5G and H), thus ruling out the possibility of an impaired lipid antigen presentation capacity of these cells.

Taken together, these data indicated that mLPA-specific T cells recognize malignant cells more efficiently than normal primary hematopoietic cells.

### Accumulation of mLPA in leukemic cells

The inventors next quantified the amount of mLPA present in primary leukemia blasts and in acute leukemia cell lines. C16 mLPA was detected in all the primary leukemia samples tested (11 AML, 5 B-ALL) (range 0.58 - 35.22 ng/106 cells), in THP1 and MOLT-4 cells (31.68 and 8.76 ng/106 cells, respectively) (Fig. 5 I), thereby confirming that mLPA is commonly present in leukemic cells. C16 mLPA was also detected in large amounts in Mo-DCs (range 0.35 - 2.96 ng/106 cells), in agreement with the stimulatory capacity of these cells (Fig. 5 E). Very low quantities of C16 mLPA were detected in normal circulating monocytes and B cells (range 0.03 - 0.07 and 0.01 - 0.05 ng/106 cells, respectively) (Fig. 5 I), in agreement with their poor T cell stimulatory activity (Fig. 5D and F).

These data suggested that mLPA accumulates in leukemia cells while is 13 poorly present in normal hematopoietic cells with exception of *in vitro* differentiated Mo-DCs.

### mLPA-reactive T cells kill leukemic cells and limit tumor growth in vivo

To assess the relevance of leukemia recognition by mLPA-specific T cells, the inventors investigated the ability of these cells to restrict the growth of CD1c+ acute leukemia cells in different settings. T cell clones specific for mLPA were capable ofkilling CD1c+ primary AML and B-ALL blasts and acute leukemia cell lines in a CD1c-dependent manner *in vitro* (Fig. 6 A-C). mLPA-specific T cell clones did not kill normal B cells, unlike leukemic B cells, whereas they killed normal monocytes, albeit less efficiently than AML blasts (Fig. 6 B).

Remarkably, the mLPA specific T cells efficiently killed the CD34+ CD117+ myeloid leukemia blasts identified by the expression of the specific leukemiaassociated immune phenotype (Vidriales et al., 2003), which are responsible for the Minimal Residual Disease in treated patients (Fig. 6 D). The control *M. tuberculosis-specific* CD1c-restricted T cell clone failed to kill acute leukemia primary cells and cell lines, but efficiently killed *M. tuberculosis-*infected target cells (data not shown).

In a second type of experiments the capacity of mLPA-specific T cells to control leukemia cells *in vivo* was investigated. The transfer of mLPA-specific T cells delayed the growth of CD1c+ MOLT-4 acute leukemia cells grafted into immunodeficient NOD/scid mice (Fig. 7 A), which displayed significantly increased survival compared with control groups that received either T cell with irrelevant specificity (CD1c-restricted and *M. tuberculosis-specific*) or vehicle alone (Fig. 7 B).

MOLT-4 cells were used because they express CD1c (Fig. 1 A and 6 A), contain high amounts of mLPA (Fig. 5 I) and efficiently graft in these immuno-compromised animals (data not shown). The two transferred T cell clones displayed a similar persistence *in vivo.* They both declined from an average 10% of the mouse PBMCs on day +1 after transfer to undetectable levels on day +7. The adoptive transfer of the same mLPA-specific T cell clone also significantly delayed the progression of primary CD1c+ AML blasts, purified from the peripheral blood of a patient and transferred into immuno-compromised NOD/scid/common γ chain-/- (NSG) mice (Fig. 7 C). In all the mice, MOLT-4 cells retained CD1c expression upon progression, whereas the primary AML blasts reduced CD1c expression at week +5 post-transfer independently of the transfer of mLPA-specific T cells (data not shown). Taken together these findings, indicate that *i)* the long term control of leukemia by the mLPA-specific T cells is probably ascribed to killing functions exerted in the first week after adoptive transfer; *ii)* progression of both leukemia was not linked to CD1c down-regulation as escape mechanisms; *iii)* the maintenance of CD1c-gene expression in primary AML blasts transferred in NSG mice depended upon specific environmental cues.

Collectively, these experiments underscored the capacity of mLPA-specific T cells to limit leukemia progression *in vivo,* suggesting the possibility to harness these T cells for the adoptive immunotherapy of CD1c+ acute leukemia. These results indicated that mLPA-specific T cells may be generated and expanded for adoptive immunotherapy of acute leukemia.

### Absence of CD1c expression in hematopoietic precursors and in nonhematopoietic tissues of healthy individuals

The inventors finally investigated the expression of CD1c in hematopoietic precursors and other not lymphoid solid tissues to determine whether they might be target of mLPA-specific CD1c-restricted T cells. CD1c was undetectable by flow cytometry on relevant subsets of primary CD34+ hematopoietic stem cells (HSC) and immature precursors contained in cord blood (Fig. 8 A), suggesting that HSC recognition by CD1c self-reactive T cells is unlikely to occur in healthy individuals. CD1c mRNA was also almost undetectable in several parenchymatous organs and non-lymphoid tissues (Fig. 8 B), confirming that tissues do not express CD1c (Brigl and Brenner, 2004; Porcelli and Modlin, 1999).

These results suggested that hematopoietic cell precursors as well as solid organs are unlikely to be targeted by mLPA-specific CD1c-restricted T cells.

### DISCUSSION

The tumor-associated antigens identified so far consist primarily of proteins that stimulate specific T cells only when processed into small peptides presented by HLA molecules (Seremet et al., 2011; Speiser and Romero, 2010). In this study, the inventors identified a unique class of lipid self-antigens, exemplified by mLPA, which may represent promising novel targets for immunotherapy in human leukemia. The inventors readily detected mLPA in primary human leukemia cells, and confirmed that this antigen is potently stimulatory for CD1c-restricted T cell clones, which restrained leukemia progression in mice xenografted with human tumor cells. mLPA is an unusual lysoglycerophosphate molecule never described before. It includes at least two different alkyl chains and displays two prominent structural features. The first is the presence of a methyl group on the phosphate moiety, which is shared in common with lysophosphomethanol (LPM). Both LPM and mLPA are methylated lysophosphatidic acids (LPA), but where LPM incorporates a *sn*-1 ester bonded alkyl chain, mLPA instead features an ether bond. LPM is generated by autotaxin-mediated cleavage of lysophosphatidylcholine and accumulates in mouse cells to promote the proliferation and migration of tumors (Endo et al., 2009). The close structural similarity of mLPA and LPM suggests that these molecules could exert similar biological effects in tumor cells.

The second prominent structural feature of mLPA is the ether bond that links the alkyl chain to glycerol, which is a peroxisomal hallmark of ether-bonded lipid synthesis (Wanders et al., 2010). Peroxisome numbers increase in tumor cells (Cha et al., 2009) and increased beta oxidation of fatty acid in peroxisomes is critical for tumor cell growth (Schrader and Fahimi, 2006). Since augmented peroxisome frequency and activity are characteristic of altered tumor cell metabolism, it is conceivable that the immune system recognizes accumulated peroxisome-derived lipids as tumor antigens. Indeed, a recent study has reported that a peroxisome-derived lipid antigen promotes thymic expansion and peripheral homeostasis of natural killer T cells (Facciotti et al., 2012). Peroxisomes thus represent an important source of lipids that can stimulate specific T cell responses in immune-regulation and tumor surveillance, and may therefore also contribute to host protection against leukemias. A key finding of the inventors' study was that mLPA can be detected in large amounts in different types of primary leukemic blasts and in established tumor cell lines of different hematopoietic lineages. The inventors also measured high levels of mLPA in Mo-DCs, induced upon culturing with the inflammatory cytokines GM-CSF and IL-4, whereas it was detected at trace levels in normal circulating monocytes and B cells, consistent with their different stimulatory activity for T cells. In this respect, the cellular distribution of mLPA resembles that of other tumor-associated antigens that accumulate in tumor cells but are also present in healthy cells (Boon et al., 2006; Jager et al., 2001). Lysophosphatidylcholine, another lysophosphoglycerolipid, was also found to accumulate in the plasma in myeloma patients and to stimulate CD1drestricted Type II NKT cells. These latter cells were proposed to regulate chronic inflammation in cancer patients (Chang et al., 2008). These data support a similar link between oncogenetic stress, lysophospholipid biosynthesis and CD1-restricted T cell responses.

Lipolytic release and remodeling of free fatty acids are associated with metabolic changes in tumor cells (Hsu and Sabatini, 2008; Vander Heiden et al., 2009), and could contribute to mLPA accumulation in cancers. Tumor cells often express increased levels of monoacylglycerol lipase, an enzyme that also generates lysophospholipids (Nomura et al., 2010) and induces accumulation of LPA. LPA is structurally similar to mLPA and can rescue cancer cells from migration defects and promote oncogenesis via activation of G protein-coupled receptors (Dorsam and Gutkind, 2007). LPA also promotes cytoskeletal reorganization, chemotaxis, and cell growth (Mills and Moolenaar, 2003), as well as inducing CXCL1 secretion, tumor progression, vascular remodeling and reversal of cell differentiation (reviewed in (van Meeteren and Moolenaar, 2007). mLPA may confer similar advantages on rapidly growing cells, which could explain the accumulation of this lipid in tumors.

The inventors' findings raise important questions concerning the physiological role of mLPA-specific T cells in healthy individuals vs. cancer patients. This particular antigen specificity might 'jump-start' immune responses by promoting rapid DC maturation and accelerating the expansion of antigen-specific B and T cells (Cerundolo et al., 2009; Vincent et al., 2002). Recognition of accumulating mLPA might represent a mechanism by which the host immune system recognizes self-lipid molecules as markers of cellular stress (De Libero et al., 2005). In addition, mLPA-specific T cells may participate in tumor immune-surveillance, using their cytolytic capacity to eliminate potentially tumorigenic cells undergoing uncontrolled proliferation. Further investigation is warranted to unravel whether in patients the extent of the leukemia burden might have reduced T cells involved in leukemia immune surveillance. The value of mLPA-specific T cells as novel immunotherapeutic tools is supported by their protective effect in a mouse xenograft model of human leukemia.

These experiments suggested that mLPA-specific T cells may target leukemic cells in human patients *in vivo.* Adoptive T cell immunotherapy strategies that selectively target malignant cells without inducing GVHD hold promise for the control of tumor recurrence after HSCT in acute leukemia (Kolb, 2008). Targeting CD1c molecules and tumor-associated lipid antigens for immunotherapy might ideally integrate strategies that target protein antigens with remarkable advantages. Unlike peptide antigens (Matsushita et al., 2012), lipid antigens do not undergo structural changes when subject to the strong selective pressure of specific immune responses.

Alterations of entire lipid biosynthetic pathways are often incompatible with cell survival, thus making loss of lipid antigens in tumor cells an unlikely mechanism of cancer immune evasion. Furthermore, the non-polymorphic nature of CD1c and high frequency of CD1c+ leukemia (∼50%) might make adoptive immunotherapy with mLPA-specific T cells applicable in a large number of patients. The adoptive transfer of mLPA-specific T cells might complement immunotherapy with HLA-restricted T cells in HLA-haploidentical transplants, in which the leukemia blasts relapse and escape immune recognition after loss of HLA (Vago et al., 2009). An issue that deserves careful evaluation is that mLPA-specific T cells killed normal monocytes *in vitro.* In the case of mLPA-specific adoptive T cell therapy in the course of HSCT, this side effect might be tolerated by patients during the peritransplant period, when the control of minimal residual leukemia is paramount.

Importantly, the absence of CD1c in hematopoietic cell precursors, and its almost undetectable expression in parenchymatous organs and non-lymphoid tissues might prevent harmful reactions of transferred mLPA-specific T cells. Transduced cells might also be engineered with suicide genes, currently utilized in the clinics (Ciceri et al., 2009), to prevent or halt GVHD.

In conclusion, the inventors have identified mLPA as novel lipid antigen that potently activates CD1c-restricted human T cells. The broad expression of CD1 molecules by a large number of primary acute leukemias, combined with the wide distribution of mLPA in leukemias and the presence of mLPA-specific T cells in blood of healthy individuals, could provide new opportunities for cellular immunotherapy in human patients.

### References

Amprey, J.L., J.S. Im, S.J. Turco, H.W. Murray, P.A. Illarionov, G.S. Besra, S.A. Porcelli, and G.F. Spath. 2004. JExp Med 200:895-904.
Berzofsky, J.A., and M. Terabe. 2009. Curr Mol Med 9:667-672.
Bondanza, A., V. Valtolina, Z. Magnani, M. Ponzoni, K. Fleischhauer, M. Bonyhadi, C. Traversari, F. Sanvito, S. Toma, M. Radrizzani, S. La Seta-Catamancio, F. Ciceri, C. Bordignon, and C. Bonini. 2006. Blood 107:1828-1836.
Boon, T., P.G. Coulie, B.J. Van den Eynde, and P. van der Bruggen. 2006. Annual review of immunology 24:175-208.
Brigl, M., and M.B. Brenner. 2004. Annual review of immunology 22:817-890.
Canderan, G., P. Gruarin, D. Montagna, R. Fontana, G. Campi, G. Melloni, C. Traversari, P. Dellabona, and G. Casorati. 2010. PLoS One 5:e12014.
Cerundolo, V., J.D. Silk, S.H. Masri, and M. Salio. 2009. Nat Rev Immunol 9:28-38.
Cha, M.K., K.H. Suh, and I.H. Kim. 2009. JExp Clin Cancer Res 28:93.
Chang, D.H., H. Deng, P. Matthews, J. Krasovsky, G. Ragupathi, R. Spisek, A. Mazumder, D.H. Vesole, S. Jagannath, and M.V. Dhodapkar. 2008. Blood 112:1308-1316.
Ciceri, F., C. Bonini, M.T. Stanghellini, A. Bondanza, C. Traversari, M. Salomoni, L. Turchetto, S. Colombi, M. Bernardi, J. Peccatori, A. Pescarollo, P. Servida, Z. Magnani, S.K. Perna, V. Valtolina, F. Crippa, L. Callegaro, E. Spoldi, R. Crocchiolo, K. Fleischhauer, M. Ponzoni, L. Vago, S. Rossini, A. Santoro, E. Todisco, J. Apperley, E. Olavarria, S. Slavin, E.M. Weissinger, A. Ganser, M. Stadler, E. 33
Yannaki, A. Fassas, A. Anagnostopoulos, M. Bregni, C.G. Stampino, P. Bruzzi, and C. Bordignon. 2009. The lancet oncology 10:489-500. de Jong, A., V. Pena-Cruz, T.Y. Cheng, R.A. Clark, I. Van Rhijn, and D.B. Moody. 2010. Nat Immunol 11:1102-1109.
de Lalla, C., M. Lepore, F.M. Piccolo, A. Rinaldi, A. Scelfo, C. Garavaglia, L. Mori, G. De Libero, P. Dellabona, and G. Casorati. 2011. Eur J Immunol 41:602-6 10.
De Libero, G., A.P. Moran, H.J. Gober, E. Rossy, A. Shamshiev, O. Chelnokova, Z. Mazorra, S. Vendetti, A. Sacchi, M.M. Prendergast, S. Sansano, A. Tonevitsky, R. Landmann, and L. Mori. 2005. Immunity 22:763-772.
Dellabona, P., G. Casorati, B. Friedli, L. Angman, F. Sallusto, A. Tunnacliffe, E. Roosneek, and A. Lanzavecchia. 1993. J Exp Med 177:1763-1771.
Dhodapkar, M.V., and J. Richter. 2011. Clin Immunol 140:160-166.
Dohner, H., E.H. Estey, S. Amadori, F.R. Appelbaum, T. Buchner, A.K. Burnett, H. Dombret, P. Fenaux, D. Grimwade, R.A. Larson, F. Lo-Coco, T. Naoe, D. Niederwieser, G.J. Ossenkoppele, M.A. Sanz, J. Sierra, M.S. Tallman, B. Lowenberg, and C.D. Bloomfield. 2010. Blood 115:453-474.
Dorsam, R.T., and J.S. Gutkind. 2007. Nat Rev Cancer 7:79-94.
Doulatov, S., F. Notta, E. Laurenti, and J.E. Dick. 2012. Cell stem cell 10:120-136.
Endo, T., K. Kano, R. Motoki, K. Hama, S. Okudaira, M. Ishida, H. Ogiso, M. Tanaka, N. Matsuki, R. Taguchi, M. Kanai, M. Shibasaki, H. Arai, and J. Aoki. 2009. J Biochem 146:283-293.
Facciotti, F., G.S. Ramanjaneyulu, M. Lepore, S. Sansano, M. Cavallari, M. Kistowska, S. Forss-Petter, G. Ni, A. Colone, A. Singhal, J. Berger, C. Xia, L. Mori, and G. De Libero. 2012. Nat Immunol 13:474-480.
Garcia-Alles, L.F., K. Versluis, L. Maveyraud, A.T. Vallina, S. Sansano, N.F. Bello, H.J. Gober, V. Guillet, H. de la Salle, G. Puzo, L. Mori, A.J. Heck, G. De Libero, and L. Mourey. 2006. EMBO J 25:3684-3692.
Gilleron, M., S. Stenger, Z. Mazorra, F. Wittke, S. Mariotti, G. Bohmer, J. Prandi, L. Mori, G. Puzo, and G. De Libero. 2004. J Exp Med 199:649-659.
Gumperz, J.E., C. Roy, A. Makowska, D. Lum, M. Sugita, T. Podrebarac, Y. Koezuka, S.A. Porcelli, S. Cardell, M.B. Brenner, and S.M. Behar. 2000. Immunity 12:211-221.
Han, X., and R.W. Gross. 1996. Journal of American chemical Society 118:451-457.
Hsu, F.F., and J. Turk. 2007. J Am Soc Mass Spectrum 18:2065-2073.
Hsu, P.P., and D.M. Sabatini. 2008. Cell 134:703-707.
Jager, D., E. Jager, and A. Knuth. 2001. Journal of clinical pathology 54:669-674.
Jedema, I., N.M. van der Werff, R.M. Barge, R. Willemze, and J.H. Falkenburg. 2004. Blood 103:2677-2682.
Kinjo, Y., D. Wu, G. Kim, G.W. Xing, M.A. Poles, D.D. Ho, M. Tsuji, K. Kawahara, C.H. Wong, and M. Kronenberg. 2005. Nature 434:520-525.
Kolb, H.J. 2008. Blood 112:4371-4383.
Manolova, V., Y. Hirabayashi, L. Mori, and G. De Libero. 2003. Eur J Immunol 33:29-37. Matsushita, H., M.D. Vesely, D.C. Koboldt, C.G. Rickert, R. Uppaluri, V.J. Magrini, C.D. Arthur, J.M. White, Y.S. Chen, L.K. Shea, J. Hundal, M.C. Wendl, R. Demeter, T. Wylie, J.P. Allison, M.J. Smyth, L.J. Old, E.R. Mardis, and R.D. Schreiber. 2012. Nature 482:400-404. Mattner, J., K.L. Debord, N. Ismail, R.D. Goff, C. Cantu, 3rd, D. Zhou, P. Saint-Mezard, V. Wang, Y. Gao, N. Yin, K. Hoebe, O. Schneewind, D. Walker, B. Beutler, L. Teyton, P.B. Savage, and A. Bendelac. 2005. Nature 434:525-529.
Metelitsa, L.S. 2011. Clin Immunol 140:119-129.
Mills, G.B., and W.H. Moolenaar. 2003. Nat Rev Cancer 3:582-591.
Montagna, D., R. Maccario, F. Locatelli, E. Montini, S. Pagani, F. Bonetti, L. Daudt, I. Turin, D. Lisini, C. Garavaglia, P. Dellabona, and G. Casorati. 2006. Blood 108:3843-3850.
Montamat-Sicotte, D.J., K.A. Millington, C.R. Willcox, S. Hingley-Wilson, S. Hackforth, J. Innes, O.M. Kon, D.A. Lammas, D.E. Minnikin, G.S. Besra, B.E. Willcox, and A. Lalvani. 2011. J Clin Invest 121:2493-2503.
Moody, D.B., T. Ulrichs, W. Muhlecker, D.C. Young, S.S. Gurcha, E. Grant, J.P. Rosat, M.B. Brenner, C.E. Costello, G.S. Besra, and S.A. Porcelli. 2000. Nature 404:884-888.
Moody, D.B., D.C. Young, T.Y. Cheng, J.P. Rosat, C. Roura-Mir, P.B. O'Connor, D.M. Zajonc, A. Walz, M.J. Miller, S.B. Levery, I.A. Wilson, C.E. Costello, and M.B. Brenner. 2004. Science 303:527-531.
Nomura, D.K., J.Z. Long, S. Niessen, H.S. Hoover, S.W. Ng, and B.F. Cravatt. 2010. Cell 140:49-61.
Nowbakht, P., M.C. Ionescu, A. Rohner, C.P. Kalberer, E. Rossy, L. Mori, D. Cosman, G. De Libero, and A. Wodnar-Filipowicz. 2005. Blood 105:3615-3622.
Porcelli, S.A., and R.L. Modlin. 1999. Annual review of immunology 17:297-329.
Provasi, E., P. Genovese, A. Lombardo, Z. Magnani, P.Q. Liu, A. Reik, V. Chu, D.E. Paschon, L. Zhang, J. Kuball, B. Camisa, A. Bondanza, G. Casorati, M. Ponzoni, F. Ciceri, C. Bordignon, P.D. Greenberg, M.C. Holmes, P.D. Gregory, L. Naldini, and C. Bonini. 2012. Nat Med 18:807-815.
Pui, C.H., W.L. Carroll, S. Meshinchi, and R.J. Arceci. 2011. Journal of clinical oncology : official journal of the American Society of Clinical Oncology 29:551-565.
Pui, C.H., M.V. Relling, and J.R. Downing. 2004. N Engl J Med 350:1535-1548.
Pulfer, M., and R.C. Murphy. 2003. Mass Spectrum Rev 22:332-364.
Rubnitz, J.E., B. Gibson, and F.O. Smith. 2008 Pediatr Clin North Am 55:21-51, ix.
Schrader, M., and H.D. Fahimi. 2006. Biochim Biophys Acta 1763:1755-1766.
Seremet, T., F. Brasseur, and P.G. Coulie. 2011. Cancer J 17:325-330.
Shamshiev, A., A. Donda, I. Carena, L. Mori, L. Kappos, and G. De Libero. 1999. Eur J Immunol 29:1667-1675.
Shamshiev, A., A. Donda, T.I. Prigozy, L. Mori, V. Chigorno, C.A. Benedict, L. Kappos, S. Sonnino, M. Kronenberg, and G. De Libero. 2000. Immunity 13:255-264.
Shamshiev, A., H.J. Gober, A. Donda, Z. Mazorra, L. Mori, and G. De Libero. 2002. J Exp Med 195:1013-1021.
Socie, G., and B.R. Blazar. 2009. Blood 114:4327-4336.
Speiser, D.E., and P. Romero. 2010. Semin Immunol 22:144-154.
Sriram, V., W. Du, J. Gervay-Hague, and R.R. Brutkiewicz. 2005. Eur J Immunol 35:1692-1701.
Vago, L., S.K. Perna, M. Zanussi, B. Mazzi, C. Barlassina, M.T. Stanghellini, N.F. Perrelli, C. Cosentino, F. Torri, A. Angius, B. Forno, M. Casucci, M. Bernardi, J. Peccatori, C. Corti, A. Bondanza, M. Ferrari, S. Rossini, M.G. Roncarolo, C. Bordignon, C. Bonini, F. Ciceri, and K. Fleischhauer. 2009. N Engl J Med 361:478-488.
van Meeteren, L.A., and W.H. Moolenaar. 2007. Prog Lipid Res 46:145-160.
Vander Heiden, M.G., L.C. Cantley, and C.B. Thompson. 2009. Science 324:1029-1033.
Vidriales, M.B., J.J. Perez, M.C. Lopez-Berges, N. Gutierrez, J. Ciudad, P. Lucio, L. Vazquez, R. Garcia-Sanz, M.C. del Canizo, J. Fernandez-Calvo, F. Ramos, M.J. Rodriguez, M.J. Calmuntia, A. Porwith, A. Orfao, and J.F. San-Miguel. 2003. Blood 101:4695-4700.
Vincent, M.S., D.S. Leslie, J.E. Gumperz, X. Xiong, E.P. Grant, and M.B. Brenner.2002. Nat Immunol 3:1163-1168.
Vincent, M.S., X. Xiong, E.P. Grant, W. Peng, and M.B. Brenner. 2005. J Immunol 175:6344-6351.
Wanders, R.J., S. Ferdinandusse, P. Brites, and S. Kemp. 2010. Biochim Biophys Acta 1801:272-280.
Wingard, J.R., N.S. Majhail, R. Brazauskas, Z. Wang, K.A. Sobocinski, D. Jacobsohn, M.L. Sorror, M.M. Horowitz, B. Bolwell, J.D. Rizzo, and G. Socie. 2011. J Clin Oncol 29:2230-2239.
Wu, D., G.W. Xing, M.A. Poles, A. Horowitz, Y. Kinjo, B. Sullivan, V. Bodmer-Narkevitch, O. Plettenburg, M. Kronenberg, M. Tsuji, D.D. Ho, and C.H. Wong. 2005. Proc Natl Acad Sci USA 102:1351-1356.
Wu, D.Y., N.H. Segal, S. Sidobre, M. Kronenberg, and P.B. Chapman. 2003. J Exp Med 198:173-181.
Zhao, Z., and Y. Xu. 2010. J Lipid Res 51:652-659.

## Claims

1. An agonist of CD1c-restricted T cells or an agonist-specific-CD1c-restricted T cell for use in the treatment and/or prevention of a hematological disorder.

2. The agonist for use according to claim 1 wherein the agonist is a self-lipid antigen.

3. The agonist for use according to claim 1 or 2 wherein the agonist is a lysoglycerolphosphate molecule.

4. The agonist for use according to any one of previous claim wherein the agonist is a Methyl-lysophosphatidic acid (mLPA), preferably C16-methyl-lysophosphatidic acid (C16-mLPA) or C18-methyl-lysophosphatidic acid (C18-mLPA).

5. The agonist for use according to any one of previous claim wherein the agonist is used as a vaccine.

6. The agonist for use according to any one of previous claim wherein the hematological disorder is **characterized by** blood and bone marrow accumulation of immature and abnormal cells derived from hematopoietic precursors.

7. The agonist for use according to any one of previous claim wherein the hematological disorder is acute leukemia, preferably primary acute myeloid leukemia or B-cell acute leukemia.

8. A pharmaceutical composition comprising an effective amount of at least one agonist according to any one of claim 1 to 7 and pharmaceutical acceptable vehicle for use in the treatment and/or prevention of a hematological disorder.

9. The pharmaceutical composition according to claim 8 further comprising an effective amount of at least another therapeutic agent.

10. The pharmaceutical composition according to claim 9 wherein the other therapeutic agent is selected from the group of: NY-ESO1, MAGEA3, WT-1, MelanA/MART1, NA-17.

11. The agonist of CD1c-restricted T cells or the agonist-secific-CD1c-restricted T cell for use according to any one of previous claim wherein said agonist or said T cell is combined with another therapeutic intervention.

12. The agonist of CD1c-restricted T cells or the agonist-secific-CD1c-restricted T cell for use according to claim 11 wherein the therapeutic intervention is allogenic hematopoietic stem cell transplantation.

13. Use of an agonist as defined in any one of claims 1 to 4 to stimulate CD1c-restricted T cell.

14. A CD1c-restricted T cell stimulated by an agonist as defined in any one of claims 1 to 4 for use in the treatment and/or prevention of a hematological disorder.

15. A method for diagnosing and/or monitoring the course of a hematological disorder and/or for assessing the efficacy of a treatment of a hematological disorder in a subject comprising:
- measuring the amount of the agonist as defined in any one of claims 1 to 4 in a biological sample obtained from the subject; or
- measuring the number and/or the frequency of an agonist-specific-CD1c-restricted T cell as defined in any one of claims 1 to 4 in a biological sample obtained from the subject.


SEQUENCE LISTING

<110>  Ospedale San Raffaele Srl
       Universitatsspital Basel
       Fondazione Centro San Raffaele

<120>  AGONIST AND USES THEREOF

<130>  BE 123228

<160>  4

<170>  PatentIn version 3.5

<210>  1
<211>  28
<212>  DNA
<213>  Artificial Sequence

<220>
<223>  synthetic primer

<400>  1
ctcgaggaca tgctgtttct gcagtttc                                          28


<210>  2
<211>  31
<212>  DNA
<213>  Artificial Sequence

<220>
<223>  synthetic primer

<400>  2
gcggccgctc acaggatgtc ctgatatgag c                                      31


<210>  3
<211>  28
<212>  DNA
<213>  Artificial Sequence

<220>
<223>  synthetic primer

<400>  3
ctcgaggaca tgctgtttct gcagtttc                                          28


<210>  4
<211>  28
<212>  DNA
<213>  Artificial Sequence

<220>
<223>  synthetic primer

<400>  4
ccatcgatgg ggtttctcca gtagagga                                          28